(19)

Europäisches
Patentamt
European
Patent Office
Office européen
des brevets

(11) **EP 3 878 474 A1**

(12) **EUROPEAN PATENT APPLICATION**
published in accordance with Art. 153(4) EPC

(43) Date of publication:
**15.09.2021 Bulletin 2021/37**

(51) Int Cl.:
*A61K 48/00* (2006.01)    *A61K 47/54* (2017.01)
*A61K 31/7088* (2006.01)    *A61K 9/00* (2006.01)
*A61P 17/00* (2006.01)    *A61K 8/60* (2006.01)
*A61Q 19/00* (2006.01)

(21) Application number: **19882198.5**

(22) Date of filing: **23.10.2019**

(86) International application number:
**PCT/KR2019/013970**

(87) International publication number:
**WO 2020/096234 (14.05.2020 Gazette 2020/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.11.2018 KR 20180135892**

(71) Applicant: **Seasun Therapeutics
Daejeon 34015 (KR)**

(72) Inventors:
• **YU, Ji-Yeon
Cheongju-si Chungcheongbuk-do 28193 (KR)**

• **KIM, Hye Joo
Daejeon 34080 (KR)**
• **KANG, Yusun
Daejeon, 34014 (KR)**
• **LEE, Dong In
Daejeon, 34696 (KR)**
• **PARK, Hee Kyung
Daejeon 34034 (KR)**

(74) Representative: **Schüssler, Andrea
Kanzlei Huber & Schüssler
Truderinger Strasse 246
81825 München (DE)**

(54) **COMPOSITION FOR PREVENTING OR TREATING ATOPIC DERMATITIS COMPRISING SKIN-PENETRATING NUCLEIC ACID COMPLEX AS EFFECTIVE COMPONENT**

(57) The present invention relates to a composition for preventing or treating skin diseases, comprising a skin-penetrating nucleic acid complex as an effective component, and more specifically, to a pharmaceutical composition or a cosmetic composition for preventing, alleviating, or treating atopic dermatitis, comprising a skin-penetrating nucleic acid complex in which a carrier peptide nucleic acid and a bioactive nucleic acid targeting TLR2 or IL-4Rα are connected by complementary binding. The skin-penetrating nucleic acid complex according to the present invention, in which the carrier peptide nucleic acid and the bioactive nucleic acid targeting TLR2 or IL-4Rα are connected by complementary binding, has high skin permeation and skin retention, and thus is useful in the prevention, alleviation or treatment of skin diseases such as atopic dermatitis.

Fig 7a

Skin Biopsy

NC     PC     SC     PNA

**Description**

[Technical Field]

The present invention relates to a composition for preventing or treating a skin disease containing a skin-permeable nucleic acid complex as an active ingredient. More specifically, the present invention relates to a pharmaceutical composition or a cosmetic composition for preventing, ameliorating or treating atopic dermatitis containing a skin-permeable nucleic acid complex in which a bioactive nucleic acid targeting TLR2 or IL-4Rα and a carrier peptide nucleic acid are complementarily bound to each other.

[Background Art]

Unlike traditional drugs, nucleic acid drugs inhibit the expression of target-specific messenger RNA (mRNA). Thus, research is ongoing into the use of nucleic acid drugs in areas in which treatment with conventional drugs that target proteins is impossible (Kole R. et al. Nature Rev. Drug Discov. 2012; 11; 125-140., Wilson C. et al. Curr. Opin. Chem. Bio. 2006; 10: 607-614.).

Despite the excellent effects and wide variety of application of gene expression regulation based on oligo nucleic acids, there are many obstacles to be overcome in order to develop therapeutic agents based on nucleic acids. For example, the oligonucleotides may be damaged by nucleases and the like, or may be incapable of permeating the cell membrane through passive diffusion due to the electrical properties (charges) and size thereof. In order to solve the above problems, efforts are made to secure biological stability through modification of nucleic acids. Modified artificial nucleic acids are capable of increasing affinity with target nucleic acids without loss of biological activity. Peptide nucleic acid (PNA), which is a type of modified artificial nucleic acid, is an artificial nucleic acid introduced with a (2-aminoethyl)-glycine peptide backbone and has the property of strongly binding to RNA and DNA having complementary nucleotide sequences. In particular, the peptide nucleic acids have resistance to nucleases and high biological stability. Thus, research associated with therapeutic agents based on various oligo nucleic acids is underway. However, the peptide nucleic acid is difficult to introduce into cells due to the electrical neutrality thereof (Joergensen M. et al. Oligonucleotides 2011, 21; 29-37.). The ability of oligonucleotides to permeate cell membranes is quite low. In particular, DNA or RNA is negatively charged and thus cannot pass through the hydrophobic phospholipid bilayer of cells, and is difficult to deliver to cells through simple diffusion. The use of virus carriers such as retrovirus or AAV (adeno-associated virus) enables the introduction of oligonucleotides into cells. However, there are risks of unintended immune activity, the possibility of oncogene recombination and the like (Couto L. B. et. al. Curr. Opin. Pharmacol. 2010, 5; 534-542.).

For this reason, the development of nucleic acid carriers based on non-viral oligonucleotides with low cytotoxicity and low immunological activity is increasingly important. As a result, methods of introducing nucleic acids using cationic lipids, liposomes, stable nucleic acid lipid particles (SNALPs), polymers, and cell-penetrating peptides are being developed (Zhi D. et al. Bioconjug. Chem. 2013, 24; 487-519., Buyens K. et al. J. Control Release, 2012, 158; 362-70., ROSSI, J. J. et al. Gene Ther. 2006, 13: 583-584., Yousefi A. et al. J. Control Release, 2013, 170; 209-18., Trabulo S. et al. Curr. Pharm. Des. 2013, 19; 2895-923.). Such a nucleic acid delivery method includes a step for forming a complex having a functional moiety based on direct binding, and has problems associated with endosomal escape efficiency and biotoxicity of the liposome structure. Thus, there is a need to improve the function of introducing oligo nucleic acids into cells and to solve problems associated with preparation procedures and side effects.

Meanwhile, the skin is the organ with the largest surface area in the human body, and serves as a passage through which drugs can be effectively delivered when using appropriate methods. Therefore, administration of a physiologically active agent, such as a therapeutic drug, through the skin, commonly referred to as "transdermal delivery", has received great interest due to characteristics such as relatively simple administration regimen of drugs etc. Structurally, the skin consists of two main parts, namely, a thinner outermost layer, called the "epidermis (epidermal layer)", and a thicker inner layer, called the "dermis (dermal layer)". In particular, the outermost layer of the epidermis, called the "stratum corneum", is made up of flat dead cells filled with keratin. The area between the flat dead cells of the stratum corneum is made up of lipids that form the lamellar phase, which contributes to the natural barrier properties of the skin. The stratum corneum or the like present in the outermost region of the skin acts as a natural barrier, thus greatly reducing skin permeability of foreign substances such as therapeutic drugs and making it difficult to deliver hydrophilic substances having high molecular weights.

In order to overcome the defense mechanism of the skin barrier against foreign substances when delivering therapeutic drugs to the skin, studies on various skin permeability methods have been made. However, it is considered to be very difficult to deliver drugs based on the properties of chemical substances without physically damaging or irritating the skin, and there is demand for continued development due to the various advantages that can be expected from materials that overcome the difficulty. In response to this demand, a number of studies have been reported on skin-permeating delivery materials such as liposomes, nanoparticles, and peptide ligands that are capable of effectively delivering drugs with a large molecular weight [Lademann et al., 2007 (Eur. J. Pharm. Biopharm. 2007 May;66(2):159-64.), Chen et al., 2006a (J. Pharmacol. Exp. Ther. 2006 Nov;319(2):765-75.), Chen et al., 2006b (Nat. Biotechnol. 2006 Apr;24(4):455-60.)]. Despite the increasing costs for development into the practical application stage through this, the efficiency and stability of the drugs to be delivered cannot be guaranteed, and thus the development of materials having both a skin permeability function and *in-vivo* efficacy is required. In order to realize effectiveness of external treatment materials applied to the skin surface, a great deal of effort is required due to the technical difficulty of passing through the epidermis and the dermis.

In this regard, surprisingly, the present inventors found that a nucleic acid complex containing a bioactive nucleic acid and a carrier peptide nucleic acid modified to have an overall positive charge, which complementarily bind to each other, improved cell permeability, and very efficiently regulated the expression of the target gene using the nucleic acid complex, and thus filed a patent application with regard to a novel construct that has low cytotoxicity and improved cell permeability and gene expression regulation ability of bioactive nucleic acids (PCT/KR2017/008636).

As a result of continuously conducting research on the improvement of skin permeability and cell delivery functions of the construct and therapeutic drugs, the present inventors found that a nucleic acid complex containing a bioactive nucleic acid and a carrier peptide nucleic acid modified to have an overall positive charge, which are complementarily bound to each other, has a property of very efficiently passing through the skin, preferably the stratum corneum and/or epidermal layer, and thus has excellent skin permeability.

Accordingly, as a result of extensive efforts to apply the nucleic acid complex having excellent skin permeability to the treatment of skin diseases, the present inventors proved that the skin-permeable nucleic acid complex containing a bioactive nucleic acid targeting TLR2 or IL-4Rα and a carrier, which are complementarily bound to each other, exhibits excellent effects of preventing or treating atopic dermatitis. Based on this finding, the present invention has been completed.

[Disclosure]

It is an object of the present invention to provide a pharmaceutical composition or a cosmetic composition for preventing, ameliorating or treating skin diseases containing, as an active ingredient, a skin-permeable nucleic acid complex having high skin permeability, high skin retention and excellent preventive or therapeutic effects on atopic dermatitis, and containing a bioactive nucleic acid targeting TLR2 or IL-4Rα and a carrier, which are complementarily bound to said bioactive nucleic acid.

In accordance with an aspect of the present invention, the above and other objects can be accomplished by the provision of a pharmaceutical or cosmetic composition for preventing, ameliorating or treating skin diseases comprising, a skin-permeable nucleic acid complex containing a bioactive nucleic acid having a sequence capable of binding to a TLR2 or IL-4Rα gene; and a carrier peptide nucleic acid, which are complementarily bound to said bioactive nucleic acid as an active ingredient.

In another aspect of the present invention, provided is a formulation containing the composition.

In another aspect of the present invention, provided is a method of preventing or treating skin diseases including administering a skin-permeable nucleic acid complex containing a bioactive nucleic acid having a sequence capable of binding to a TLR2 or IL-4Rα gene and a carrier peptide nucleic acid, which are complementarily bound to said bioactive nucleic acid.

In another aspect of the present invention, provided is the use of a skin-permeable nucleic acid complex containing a bioactive nucleic acid having a sequence capable of binding to a TLR2 or IL-4Rα gene and a carrier peptide nucleic acid, which are complementarily bound to said bioactive nucleic acid for the prevention or treatment of skin diseases.

In another aspect of the present invention, provided is the use of a skin-permeable nucleic acid complex containing a bioactive nucleic acid having a sequence capable of binding to a TLR2 or IL-4Rα gene and a carrier peptide nucleic acid, which are complementarily bound to said bioactive nucleic acid for the preparation of a drug for preventing or treating skin diseases.

[Description of Drawings]

FIGS. 1A to 1C schematically illustrate the effect of a type of structure in which a bioactive nucleic acid and a carrier peptide nucleic acid are bound to each other in an atopic-dermatitis-like cell model.

FIG. 1A shows the cell viability of the type of structure in which a bioactive nucleic acid and a carrier peptide nucleic acid are bound to each other in a human-derived keratin cell line induced with atopic dermatitis using a house dust mite extract (*Dermatophagoides farina*) extract.

FIG. 1B shows the cell viability of the type of structure in which a bioactive nucleic acid and a carrier peptide nucleic acid are bound to each other in a human-derived keratin cell line induced with atopic dermatitis using a house dust mite extract (*Dermatophagoides pteronyssinus*) extract.

FIG. 1C shows the cell viability of the type of structure in which a bioactive nucleic acid and a carrier peptide nucleic acid are bound to each other in a human-derived keratin cell line induced with atopic dermatitis using a chemical (2-dinitroclorobenzene, DNCB) causing sick house syndrome.

FIGS. 2A to 2C schematically illustrate the effect of a type of structure in which a bioactive nucleic acid and a carrier peptide nucleic acid are bound to each other in an atopic-dermatitis-like cell model.

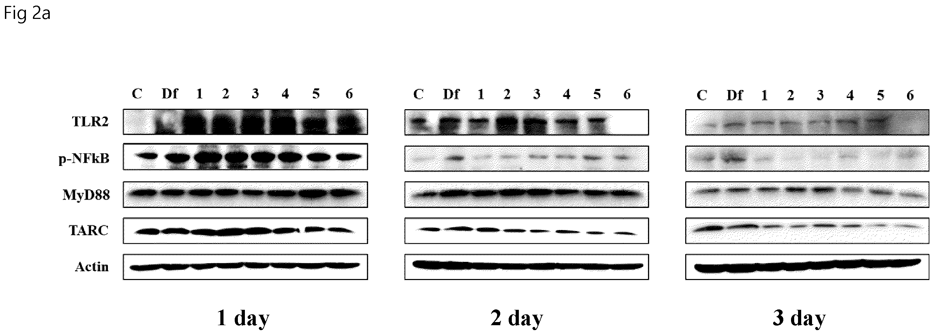

FIG. 2A shows the effect of the type of structure in which a bioactive nucleic acid and a carrier peptide nucleic acid are bound to each other on the expression of a target gene TLR2 and on downstream gene expression in a human-derived keratin cell line induced with atopic dermatitis using a *Dermatophagoides farina* extract.

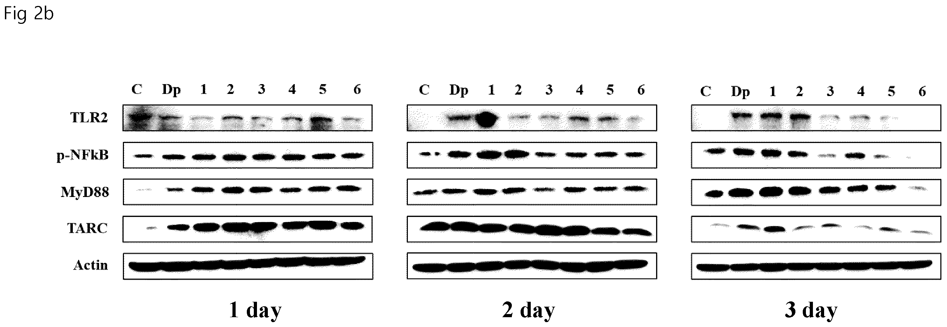

FIG. 2B shows the effect of the type of structure in which a bioactive nucleic acid and a carrier peptide nucleic acid are bound to each other on the expression of a target gene TLR2 and on the downstream gene expression in a human-derived keratin cell line induced with atopic dermatitis using a *Dermatophagoides pteronyssinus* extract.

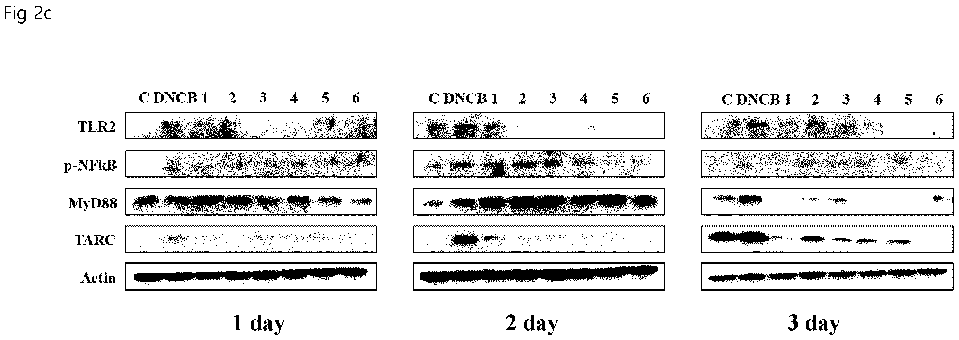

FIG. 2C shows the effect of the type of structure in which a bioactive nucleic acid and a carrier peptide nucleic acid are bound to each other on the expression of a target gene TLR2 and the downstream gene expression in a human-derived keratin cell line induced with atopic dermatitis using a chemical (2-dinitroclorobenzene, DNCB) causing sick house syndrome.

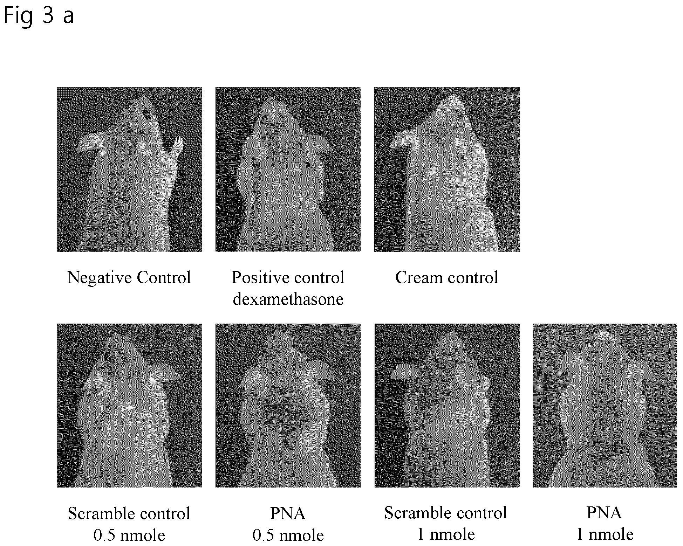

FIGS. 3A to 3H show the therapeutic effect of a nucleic acid complex targeting a TLR2 gene in an atopic-dermatitis-induced animal model.

FIG. 3A is an image showing the decrease in the atopic dermatitis phenotype of mice due to the nucleic acid complex in NC/Nga mice.

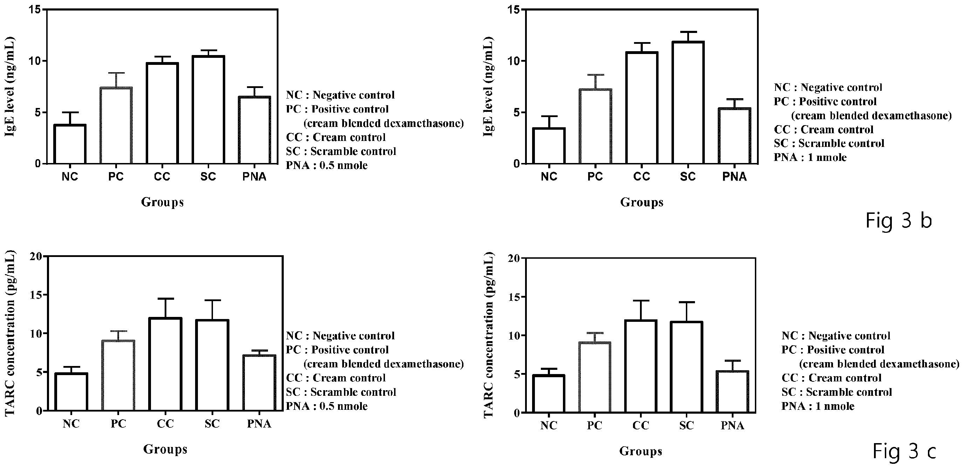

FIG. 3B shows that the concentration of IgE in the serum is decreased by the nucleic acid complex in NC/Nga mice induced with atopic dermatitis using a house dust mite extract.

FIG. 3C shows the reduction in concentration of TARC in the serum due to the nucleic acid complex in NC/Nga mice induced with atopic dermatitis using a house dust mite extract.

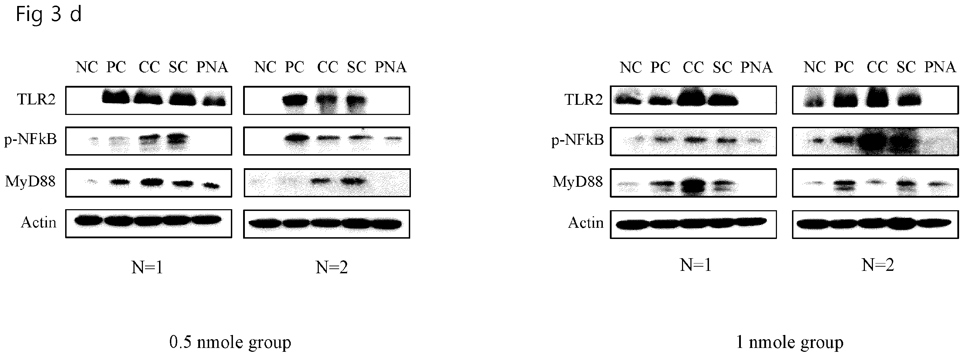

FIG. 3D shows the reduction in expression of the target gene TLR2 and downstream genes due to the nucleic acid complex in the skin tissue of NC/Nga mice induced with atopic dermatitis using a house dust mite extract.

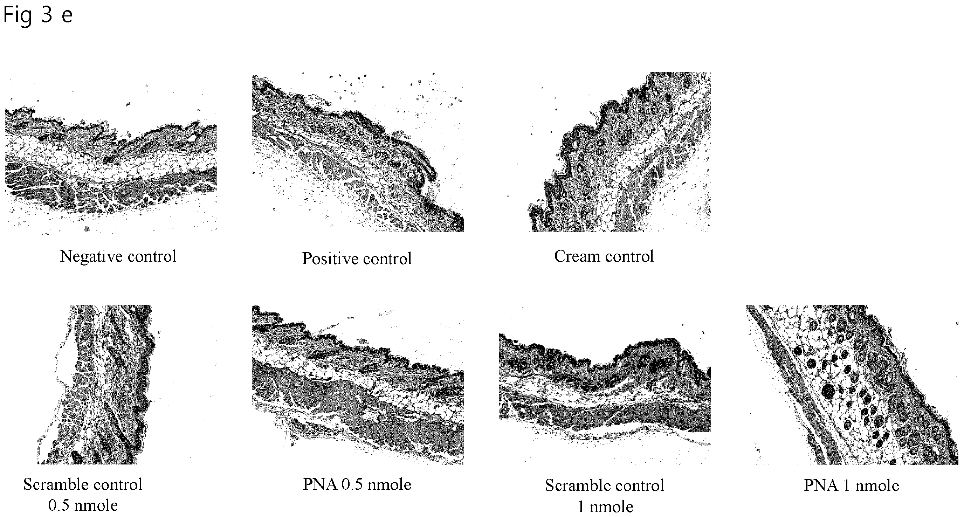

FIG. 3E shows the reduction in transdermal thickness due to the nucleic acid complex in the skin tissue of NC/Nga mice induced with atopic dermatitis using a house dust mite extract.

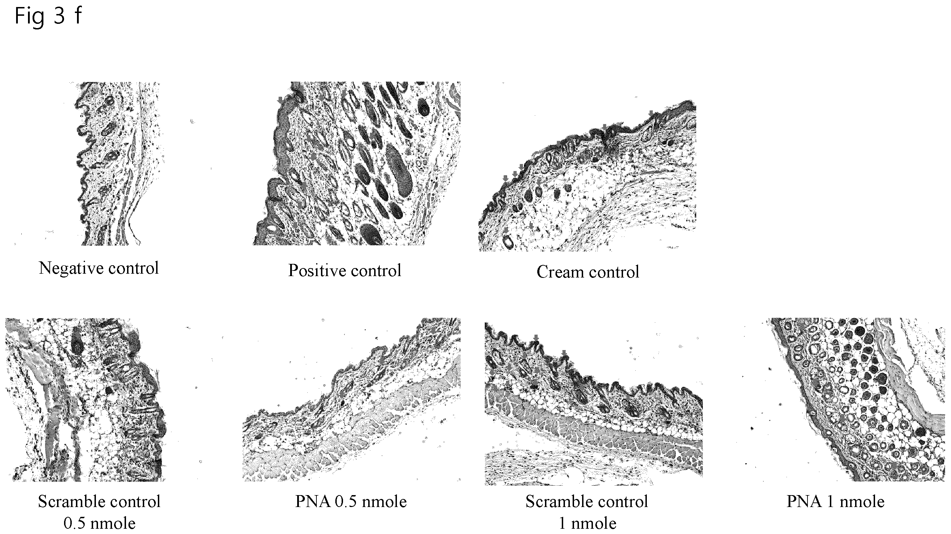

FIG. 3F shows the reduction in the inflammatory marker CD3 due to the nucleic acid complex in the skin tissue of NC/Nga mice induced with atopic dermatitis using a house dust mite extract.

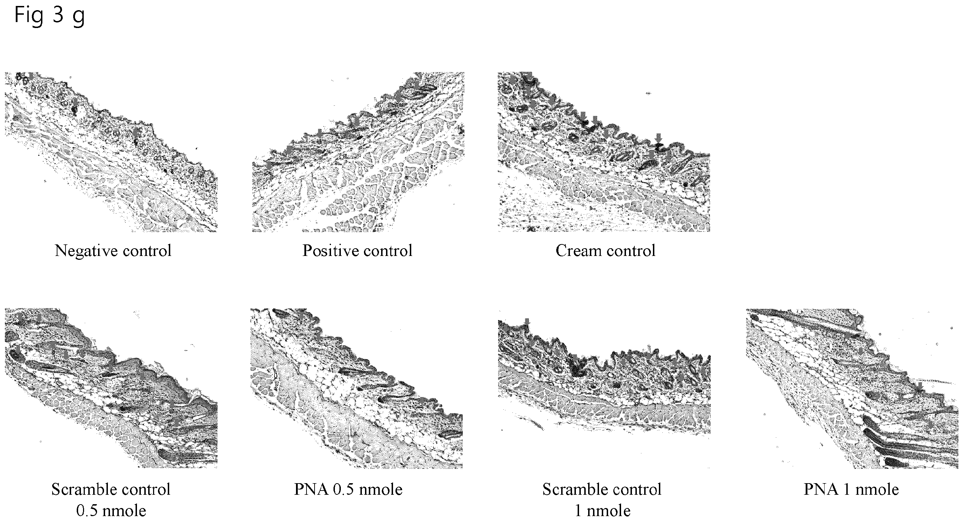

FIG. 3G shows the reduction in the inflammatory marker CDIlc due to the nucleic acid complex in the skin tissue of NC/Nga mice induced with atopic dermatitis using a house dust mite extract.

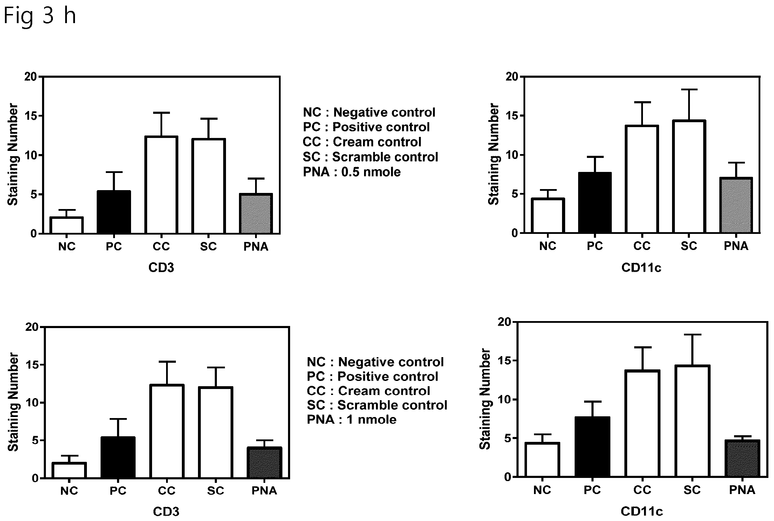

FIG. 3H shows the reduction in the inflammatory marker CD3/CD11c due to the nucleic acid complex in the skin tissue of NC/Nga mice induced with atopic dermatitis using a house dust mite extract.

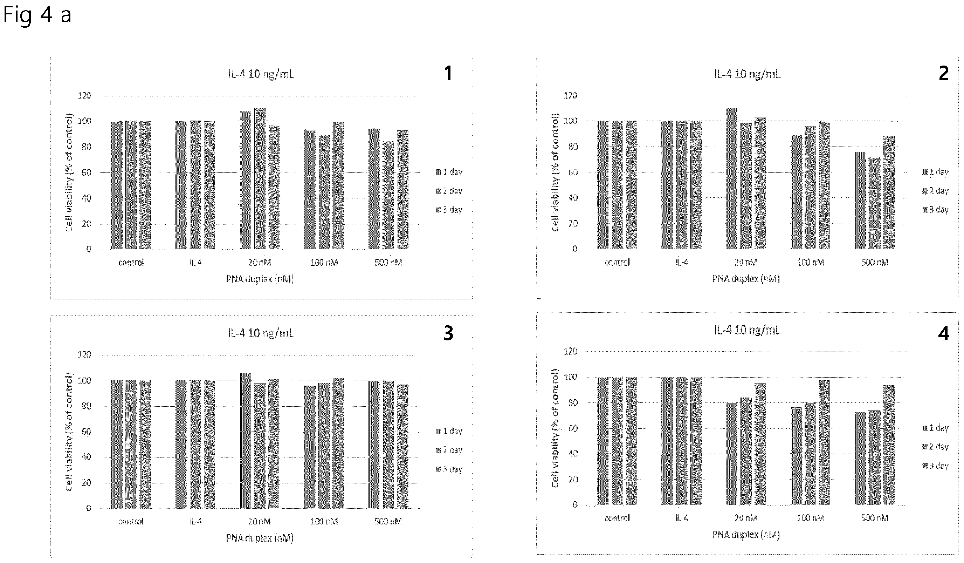

FIGS. 4A to 4B schematically illustrate the effect of the type of structure in which a bioactive nucleic acid and a carrier peptide nucleic acid are bound to each other in an atopic-dermatitis-like cell model.

FIG. 4A shows the cell viability of the type of structure in which a bioactive nucleic acid and a carrier peptide nucleic acid are bound to each other in a human-derived keratin cell line induced with atopic dermatitis using IL-4.

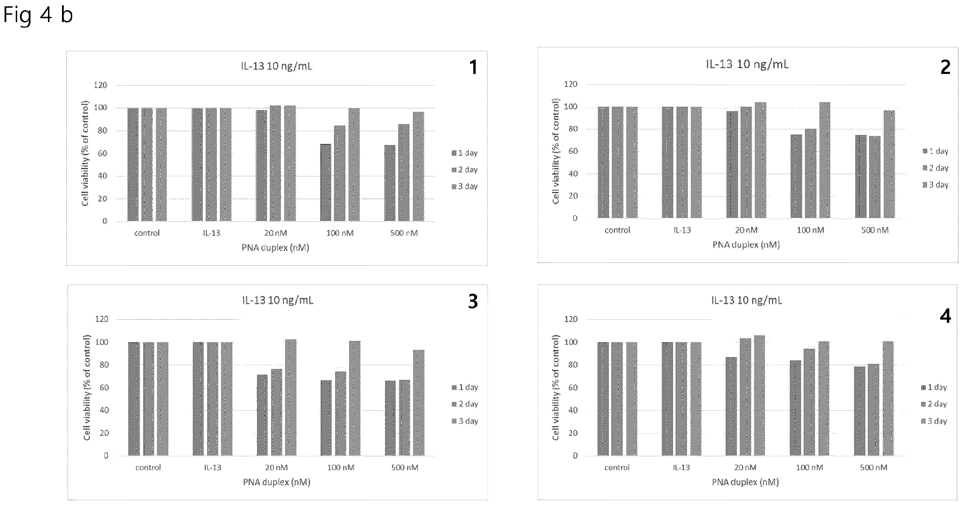

FIG. 4B shows the cell viability of the type of structure in which a bioactive nucleic acid and a carrier peptide nucleic acid are bound to each other in a human-derived keratin cell line induced with atopic dermatitis using IL-13.

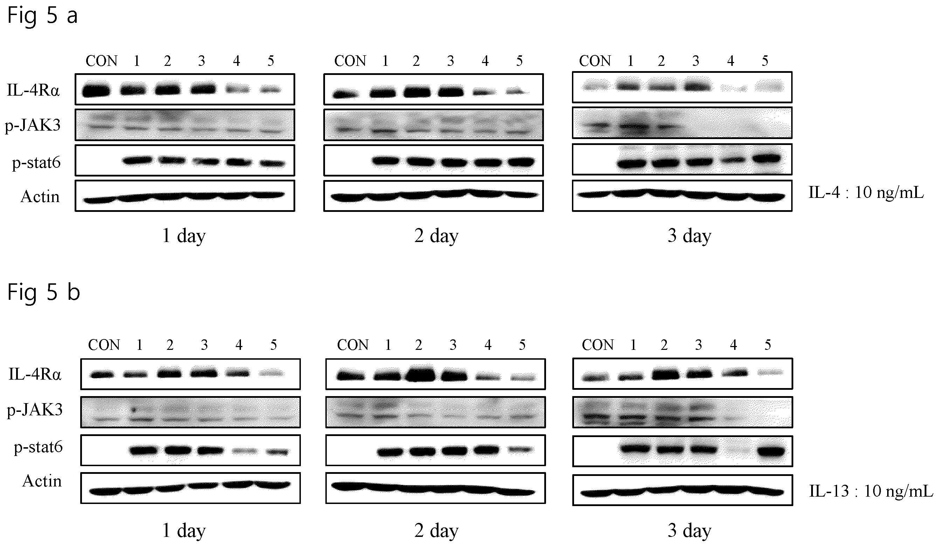

FIGS. 5A to 5B schematically illustrate the effect of the type of structure in which a bioactive nucleic acid and a carrier peptide nucleic acid are bound to each other in an atopic-dermatitis-like cell model.

FIG. 5A shows the effect of the type of structure in which a bioactive nucleic acid and a carrier peptide nucleic acid are bound to each other on the expression of a target gene IL-4R$\alpha$ and on downstream gene expression in a human-derived keratin cell line induced with atopic dermatitis using IL-4.

FIG. 5B shows the effect of the type of structure in which a bioactive nucleic acid and a carrier peptide nucleic acid are bound to each other on the expression of a target gene IL-4R$\alpha$ and on downstream gene expression in a human-derived keratin cell line induced with atopic dermatitis using IL-13.

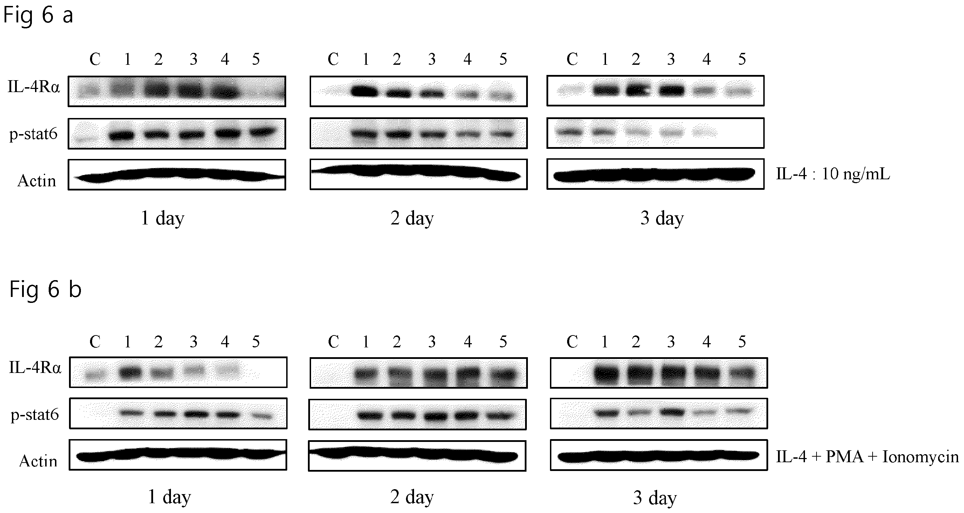

FIGS. 6A to 6B schematically illustrate the effect of the type of structure in which a bioactive nucleic acid and a carrier peptide nucleic acid are bound to each other in an atopic-dermatitis-like cell model.

FIG. 6A shows the effect of the type of structure in which a bioactive nucleic acid and a carrier peptide nucleic acid are bound to each other on the expression of a target gene IL-4R$\alpha$ and on downstream gene expression in T lymphocytes derived from a human induced with atopic dermatitis using IL-4.

FIG. 6B shows the effect of the type of structure in which a bioactive nucleic acid and a carrier peptide nucleic acid are bound to each other on the expression of target gene IL-4R$\alpha$ and on downstream gene expression in T lymphocytes derived from a human induced with atopic dermatitis using IL-4, PMA (phorbol-12-myristate-13-acetate), and ionomycin.

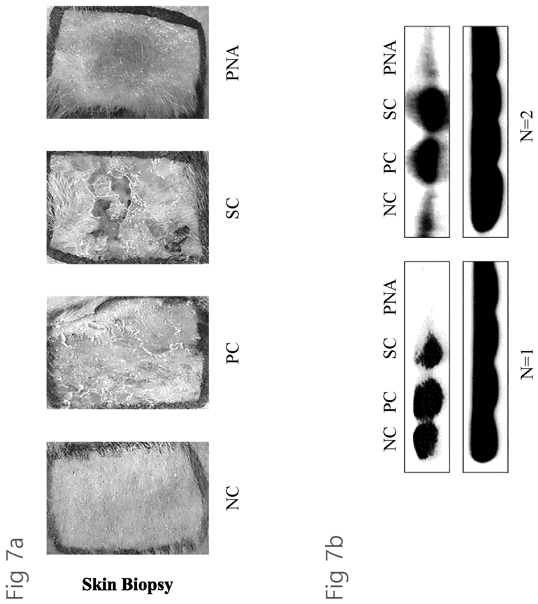

FIGS. 7A to 7B show the tissue phenotype associated with the therapeutic effect on atopic dermatitis and the inhibitory effect on protein expression in tissue by a combination of a bioactive nucleic acid and a carrier peptide nucleic acid, selected using a cell model similar to an NC/Nga mouse model induced with atopic dermatitis using a house dust mite extract.

FIG. 7A shows the improvement of skin phenotype due to the nucleic acid complex in an animal model induced with atopic dermatitis using DNCB.

FIG. 7B shows decreased expression of IL-4R$\alpha$, a target gene, in atopic-dermatitis-induced skin tissue due to the nucleic acid complex in an animal model induced with atopic dermatitis using DNCB.

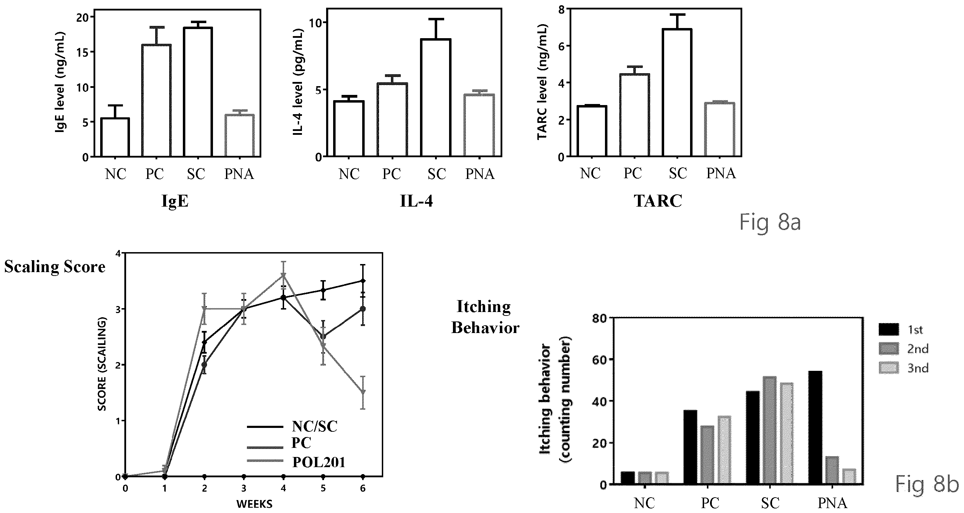

FIGS. 8A to 8B show the results of ELISA and SCORAD (atopic dermatitis behavior evaluation, pruritus and erythema) analysis showing the therapeutic effect on atopic dermatitis by a combination of a bioactive nucleic acid and a carrier peptide nucleic acid, selected using a cell model similar to an NC/Nga mouse model induced with atopic dermatitis using DNCB.

FIG. 8A shows reduced amounts of IgE, TARC and IL-4 in serum due to the nucleic acid complex in an animal model induced with atopic dermatitis using DNCB.

FIG. 8B shows amelioration of pruritus and erythema due to the nucleic acid complex in an animal model induced with atopic dermatitis using DNCB.

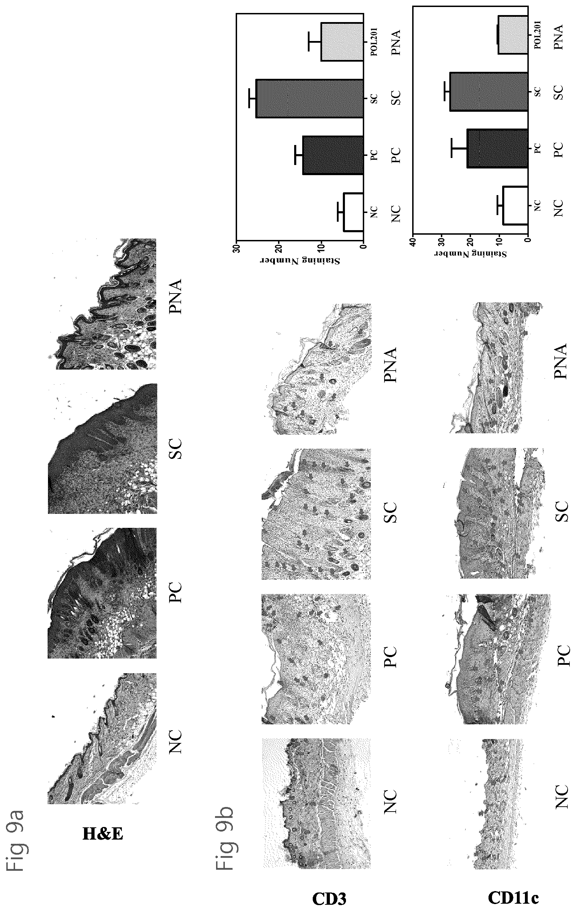

FIGS. 9A to 9B show the results of H&E staining and immunostaining of atopic dermatitis skin tissue, showing the therapeutic effect on atopic dermatitis by a combination of a bioactive nucleic acid and a carrier peptide nucleic acid, selected using a cell model similar to an NC/Nga mouse model induced with atopic dermatitis using DNCB.

FIG. 9A shows the result of H&E staining of skin tissue showing the decreased thickness of transdermal tissue and reduced expression of inflammatory cells due to the nucleic acid complex in an animal model induced with atopic dermatitis using DNCB.

FIG. 9B shows the result of immunostaining of skin tissue showing decreased expression of CD3 and CD11c, as dendritic cells and macrophage markers, in skin tissue due to the nucleic acid complex in an animal model induced with atopic dermatitis using DNCB.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

The present invention is based on the finding that a nucleic acid complex containing a bioactive nucleic acid and a carrier peptide nucleic acid, which are complementarily bound to each other, has high skin permeability and high skin retention, in particular, a nucleic acid complex containing a bioactive nucleic acid targeting TLR2 or IL-4Rα and a carrier peptide nucleic acid, which are complementarily bound to each other, can be utilized through application to the skin surface in the treatment of skin diseases such as atopic dermatitis.

In one aspect, the present invention is directed to a pharmaceutical or cosmetic composition for preventing, ameliorating or treating skin diseases comprising, a skin-permeable nucleic acid complex containing a bioactive nucleic acid having a sequence capable of binding to a TLR2 or IL-4Rα gene; and a carrier peptide nucleic acid, which are complementarily bound to said bioactive nucleic acid as an active ingredient.

In the present invention, the nucleic acid complex in which the bioactive nucleic acid and the carrier peptide are complementarily bound to each other has a structure of the following Formula (1):

$$\text{Formula (1)}$$

$$[A \equiv C^{(+)}]$$

wherein

A is a bioactive nucleic acid having a sequence capable of binding to a target gene,
C is a carrier peptide nucleic acid capable of binding to the bioactive nucleic acid,
'≡' means a complementary binding between the bioactive nucleic acid and the carrier peptide nucleic acid,
wherein the bioactive nucleic acid represented by A is negatively charged or neutral overall, and
$C^{(+)}$ means that the carrier peptide nucleic acid is positively charged overall.

The carrier peptide nucleic acid contains at least one peptide nucleic acid monomer modified to be positively charged overall.

The binding between the bioactive nucleic acid and the carrier peptide nucleic acid in the nucleic acid complex according to the present invention may be antiparallel or parallel binding.

As used herein, the term "bioactive nucleic acid" refers to a nucleic acid having a complementary sequence capable of binding to a gene that is the target of expression inhibition, in particular, a nucleic acid having a complementary sequence capable of binding to the mRNA of the gene that is the target of expression inhibition, means a nucleic acid involved in the regulation of gene expression, such as inhibiting the expression of the corresponding gene, and may be a nucleic acid having a sequence complementary to a gene that is the target of expression inhibition.

In particular, the term "bioactive nucleic acid" used herein binds to a target gene and a nucleotide sequence including the same *in vitro* or *in vivo,* and acts to inhibit the inherent functions (transcript expression or protein expression) of the corresponding gene.

Therefore, the "bioactive nucleic acid" in the present invention is preferably an antisense peptide nucleic acid of TLR2 (toll-like receptor 2) and IL-4Rα (interleukin-14 receptor α), which are target genes for atopic dermatitis, and is more preferably represented by an amino acid sequence selected from the group consisting of SEQ ID NOS: 1 to 4, but is not limited thereto.

In addition, the bioactive nucleic acid capable of binding to the TLR2 gene is preferably represented by the amino acid sequence of SEQ ID NO: 2, and the bioactive nucleic acid capable of binding to the IL-4Rα gene is preferably represented by the amino acid sequence of SEQ ID NO: 4, but is not limited thereto.

As used herein, the term "carrier peptide nucleic acid" refers to a nucleic acid, the bases of which are partly or entirely complementarily bound to the bioactive nucleic acid, to impart functionality thereto, and the carrier peptide nucleic acid used herein may be a peptide nucleic acid (PNA) or a modified nucleic acid similar thereto, and is preferably a peptide nucleic acid, but is not limited thereto.

In particular, the carrier peptide nucleic acid used herein is preferably represented by an amino acid sequence selected from the group consisting of SEQ ID NOS: 5 to 18, but is not limited thereto.

As used herein, the term "skin-permeable nucleic acid complex" refers to a complex that enables penetration of a bioactive substance into the body and ultimately into cells when brought into contact with the skin. Specifically, the skin-permeable nucleic acid complex enables a bioactive substance to pass through the stratum corneum and/or epidermal layer, which is the outermost layer of the skin, and to be delivered to the epidermal layer or the dermis layer, or enables the same to pass through the dermal layer and to be delivered into the body.

The bioactive substance may remain in the stratum corneum, epidermal layer, or dermal layer, or may also pass through the dermal layer and be delivered to the body depending on the total net charge in the skin-permeable nucleic acid complex according to the present invention and/or the number of bioactive nucleic acids and/or carrier peptide nucleic acids in the nucleic acid complex.

Accordingly, in the present invention, the nucleic acid complex may have skin retention.

In the present invention, in the skin-permeable nucleic acid complex, the bioactive nucleic acid itself may function as a therapeutic agent; that is, the complex itself may function both as a skin-permeable carrier and as a therapeutic agent.

In particular, the skin-permeable nucleic acid complex according to the present invention may be transdermally delivered into the body through the skin and then may be delivered to desired cells, and may be used in any form containing the nucleic acid complex.

In the present invention, the skin-permeable nucleic acid complex preferably contains a bioactive nucleic acid represented by the amino acid sequence of SEQ ID NO: 2 or 4 and a carrier peptide nucleic acid represented by any one amino acid sequence selected from the group consisting of SEQ ID NOS: 5 to 18, but is not limited thereto.

In addition, the binding force (melting temperature, Tm) between the bioactive nucleic acid capable of binding to the TLR2 or IL-4Rα gene and the carrier peptide nucleic acid is lower than the binding force between the bioactive nucleic acid and the TLR2 or IL-4Rα gene targeted by the bioactive nucleic acid.

In the present invention, the bioactive nucleic acid or the carrier peptide nucleic acid may be further bound with a substance that facilitates endosomal escape to the 5'-end or the 3'-end of each nucleic acid. That is, the bioactive nucleic acid or the carrier peptide nucleic acid may further contain a substance that facilitates endosomal escape of the bioactive nucleic acid and the carrier peptide nucleic acid to form the structure of the following Formula (2).

$$\text{Formula (2)}$$

$$[\text{mA} \equiv \text{mC}^{(+)}]$$

wherein

"m" means a substance that facilitates endosomal escape of the bioactive nucleic acid and the carrier peptide nucleic acid.

In the present invention, the "substance that facilitates endosomal escape" may facilitate escape of the bioactive nucleic acid from the endosomes by increasing the osmotic pressure in the endosomes or destabilizing the membrane of the endosomes. It means that the "substance that facilitates endosomal escape" enables the bioactive nucleic acid to move more efficiently and rapidly to the nucleus or cytoplasm and to meet and function with the target gene (D. W. Pack, A. S. Hoffman, S. Pun, P. S. Stayton, "Design and development of polymers for gene delivery," Nat. Rev. Drug. Discov., 4, 581-593 (2005)).

In the present invention, the substance that facilitates endosomal escape includes at least one selected from the group consisting of peptides, lipid nanoparticles, polyplex nanoparticles, polymer nanospheres, inorganic nanoparticles, cationic lipidbased nanoparticles, cationic polymers, and pH-sensitive polymers.

In the present invention, as the substance that facilitates endosomal escape, a peptide having a sequence of GLFDIIKKIAESF (SEQ ID NO: 19) may be bound to the bioactive nucleic acid via a linker, and histidine (10) may be bound to the carrier peptide nucleic acid via a linker, but the present invention is not limited thereto.

In the present invention, the lipid nanoparticles may be selected from the group consisting of lipids, phospholipids, cetyl palmitate, poloxamer 18, Tween 85, tristearin glyceride, and Tween 80.

In the present invention, the polyplex nanoparticles may be poly(amidoamine) or polyethylenimine (PEI).

In the present invention, the polymer nanospheres may be selected from the group consisting of polycaprolactone, poly(lactide-co-glycolide), polylactide, polyglycolide, poly(d,l-lactide), chitosan, and PLGA-polyethylene glycol.

In the present invention, the inorganic nanoparticles may be selected from the group consisting of $Fe_2O_3$ $Fe_3O_4$, $WO_3$ and $WO_{2.9}$.

In the present invention, the cationic lipidbased nanoparticles may be selected from the group consisting of 1-(aminoethyl)iminobis[N-(oleicylcysteinyl-1-amino-ethyl)propionamide], an N-alkylated derivative of PTA and 3,5-didodecyloxybenzamidine.

In the present invention, the cationic polymer may be selected from the group consisting of vinylpyrrolidone-N,N-dimethylaminoethyl methacrylate acid copolymer diethyl sulphate, polyisobutylene and poly(N-vinylcarbazole).

In the present invention, the pH-sensitive polymers may be selected from the group consisting of polyacids, poly(acrylic acid), poly(methacrylic acid) and hydrolyzed polyacrylamide.

In the present invention, each of the bioactive nucleic acid and the carrier peptide nucleic acid contains 2 to 50, preferably 5 to 30, more preferably 10 to 25, and most preferably 15 to 17 nucleic acid monomers.

The bioactive nucleic acid may contain a natural nucleic acid base and/or a modified nucleic acid monomer.

In the present invention, in the case where the monomer used for the bioactive nucleic acid is PNA, the monomer is called a "bioactive peptide nucleic acid". In the case where other monomers are used, they are referred to in the same manner as above.

In the present invention, the bioactive nucleic acid and the carrier peptide nucleic acid may further contain at least one functional group selected from the group consisting of phosphodiester, 2'-0-methyl, 2'-methoxy-ethyl, phosphoramidate, methylphosphonate, and phosphorothioate.

In the present invention, the carrier peptide nucleic acid may have a nucleotide sequence which is partially or entirely complementary to the bioactive nucleic acid. In particular, the carrier peptide nucleic acid may include at least one universal base, and the carrier peptide nucleic acid may be entirely composed of universal bases.

In the present invention, with regard to the electrical properties, each of the bioactive nucleic acid and the carrier peptide nucleic acid in the skin-permeable nucleic acid complex may have an overall positive charge (positive), negative charge (negative), or no charge (neutral).

The term "overall" in the expression of the electrical properties means overall electrical properties of respective charges of the bioactive nucleic acids or carrier peptide nucleic acids as viewed from the outside, not the electrical properties of individual bases. For example, even though some monomers in the bioactive nucleic acid are positive, when the number of negatively charged monomers is greater than the number of positively charged monomers, the bioactive nucleic acid is negatively charged in terms of the "overall" electrical properties. When the number of positively charged bases and/or backbones is greater than the number of negatively charged bases and/or backbones, even though some bases and/or backbone constituents in the carrier peptide nucleic acid are negatively charged, the carrier peptide nucleic acid is considered to be positively charged in terms of the "overall" electrical properties thereof.

From this perspective, the nucleic acid complex of the present invention may be considered to be positively charged overall. In the nucleic acid complex, preferably, the bioactive nucleic acid is negatively charged or neutral in terms of overall electrical properties, and the carrier peptide nucleic acid is positively charged in terms of overall electrical properties, but is not limited thereto.

In the present invention, a modified peptide nucleic acid monomer may be used to impart electrical properties to the bioactive nucleic acid and the carrier peptide nucleic acid, and the modified peptide nucleic acid monomer includes, as a positively charged carrier peptide nucleic acid, at least one positively charged nucleic acid selected from the group consisting of lysine (Lys, K), arginine (Arg, R), histidine (His, H), diamino butyric acid (DAB), ornithine (Orn), and amino acid analogs, and includes, as a negatively charged carrier peptide nucleic acid, glutamic acid (Glu, E), which is a negatively charged amino acid, or a negatively charged amino acid of an amino acid analogue.

In the present invention, the carrier peptide nucleic acid may contain at least one gamma- or alpha-backbone-modified peptide nucleic acid monomer in order for the carrier peptide nucleic acid to have an overall positive charge.

The gamma- or alpha-backbone-modified peptide nucleic acid monomer contains, in the backbone thereof, at least one positively charged amino acid selected from the group consisting of lysine (Lys, K), arginine (Arg, R), histidine (His, H), diamino butyric acid, ornithine (Orn), and amino acid analogs in order for the carrier peptide nucleic acid to have an overall positive electrical charge.

In the present invention, the peptide nucleic acid monomer having a modified nucleobase may be used, in addition to the backbone modification, for modification of the peptide nucleic acid monomer so as to impart an electrical charge thereto. Preferably, an amine, triazole or imidazole moiety may be included in the nucleobase to impart a positive electrical charge thereto, or carboxylic acid may be included in the base to impart a negative electrical charge thereto.

In the present invention, the modified peptide nucleic acid monomer of the carrier peptide nucleic acid may further include a negative charge in the backbone or nucleobase. However, preferably, the modified peptide nucleic acid monomer contains more positively charged monomers than negatively charged monomers, so the carrier peptide nucleic acid is positively charged.

Preferably, the nucleic acid complex according to the present invention is positively charged overall.

In the nucleic acid complex according to the present invention, at least one material selected from the group consisting of a hydrophobic moiety, a hydrophilic moiety, a target-antigen-specific antibody, an aptamer, and a fluorescent/luminescent marker is bound to the bioactive nucleic acid and/or the carrier peptide nucleic acid. Preferably, at least one material selected from the group consisting of a hydrophobic moiety, a hydrophilic moiety, a target-antigen-specific antibody, an aptamer and a fluorescent/luminescent marker for imaging may be bound to the carrier peptide nucleic acid.

In the present invention, the binding of at least one material selected from the group consisting of a hydrophobic moiety, a hydrophilic moiety, a target-antigen-specific antibody, an aptamer, a quencher, a fluorescent marker and a luminescent marker to the bioactive nucleic acid and/or the carrier peptide nucleic acid may be a simple covalent bond or a covalent bond mediated by a linker, but is not limited thereto. Preferably, cell permeation, solubility, stability, transportation and imaging-related substances (e.g., hydrophobic residues and the like) bound to the nucleic acid carrier exist independently of the bioactive nucleic acid that regulates the expression of the target gene.

In the present invention, as described above, the complementary binding of the bioactive nucleic acid and the carrier peptide nucleic acid is generally antiparallel binding or parallel binding. Complementary binding forms a structure that is separated in the presence of the target sequence of the bioactive nucleic acid (a sequence complementary to the bioactive nucleic acid) .

The antiparallel binding and parallel binding are determined depending on the 5'-direction and the 3'-direction in the binding mode of DNA-DNA or DNA-PNA. Antiparallel binding is a general binding mode of DNA-DNA or DNA-PNA. For example, in the nucleic acid complex according to the present invention, the bioactive nucleic acid in a 5' to 3' direction is bound to the carrier peptide nucleic acid in a 3' to 5' direction. Parallel binding has a slightly smaller binding force than that of antiparallel binding, and the bioactive nucleic acid and the carrier peptide nucleic acid are bound to each other in the same direction, that is, the 5' to 3' direction or the 3' to 5' direction.

In the nucleic acid complex according to the present invention, preferably, the binding force between the bioactive nucleic acid and the carrier peptide nucleic acid is smaller than the binding force between the bioactive nucleic acid and the target gene targeted by the bioactive nucleic acid, particularly the mRNA of the target gene. The bonding force is determined by the melting temperature (Tm).

Examples of specific methods for lowering the binding force (melting temperature, Tm) between the bioactive nucleic acid and the carrier peptide nucleic acid than the binding force between the bioactive nucleic acid and the target gene targeted by the bioactive nucleic acid, particularly the mRNA of the target gene, include parallel binding or partial specific binding between the bioactive nucleic acid and the carrier peptide nucleic acid, but are not limited thereto.

As another example, the carrier peptide nucleic acid has at least one peptide nucleic acid base selected from the group consisting of a linker, a universal base, and a peptide nucleic acid base including a base that is not complementary to a corresponding base of the bioactive nucleic acid, but is not limited thereto.

In the present invention, the universal base is non-selectively bound to a natural base such as adenine, guanine, cytosine, thymine or uracil, and may include, as a base having a binding force lower than the complementary binding force, at least one selected from the group consisting of inosine PNA, indole PNA, nitroindole PNA, and abasic, and is preferably inosine PNA.

The present invention provides a combination of the binding mode and electrical properties of nucleic acids for controlling the function of the nucleic acid complex, and controls the particle size and the action time using the combination of the binding mode and electrical properties of the nucleic acids, and improves cell permeability, solubility and specificity.

In the present invention, the time point at which the bioactive peptide nucleic acid is bound to the target sequence (such as the time point at which the bioactive nucleic acid is substituted with the target sequence, and the time point at which target-specific release and binding occur) can be controlled in the presence of the target gene through control of the binding force between the bioactive peptide nucleic acid and the carrier peptide nucleic acid.

In the nucleic acid complex according to the present invention, the control of the time point of substitution (strand displacement) of the bioactive nucleic acid with the target gene and the time point of target-specific release and binding is made possible by the presence, number, and position of non-specific bases, universal bases and linkers of carrier nucleic acids for non-specific binding of the complex. The control is possible due to the combination of the factors described above and parallel or antiparallel binding, which are complementary binding modes of the peptide complex.

In the present invention, the particles of the nucleic acid complex may have a size of 5 nm to 300 nm, preferably 10 nm to 80 nm, and most preferably 15 nm to 70 nm.

In the present invention, the particle size of the nucleic acid complex may be controlled by adjusting the charge balance between the bioactive peptide nucleic acid and the carrier peptide nucleic acid. Specifically, when the positive charge of the carrier peptide nucleic acid increases, the size of the particles decreases, whereas, when the positive charge of the carrier peptide nucleic acid exceeds a certain level, the size of the particles increases. In addition, the particle size is determined by, as another important factor determining the particle size, an appropriate charge balance with the overall carrier peptide nucleic acid according to the charge of the bioactive peptide nucleic acid forming the complex.

The number of positive charges of the carrier peptide nucleic acid according to the present invention is 1 to 7 (meaning that 1 to 7 positively charged monomers are included), preferably 2 to 5, and most preferably 2 to 3, and the bioactive nucleic acid has, as a net charge balance, 0 to 5 negative charges, and preferably 0 to 3 negative charges.

In the present invention, the nucleic acid complex may be prepared by hybridizing the bioactive nucleic acid with the carrier peptide nucleic acid under appropriate conditions.

The term "hybridization" as used herein means that complementary single-stranded nucleic acids form double-stranded nucleic acids. Hybridization may occur when the complementarity between the two nucleic acid strands is a perfect match or when some mismatched bases are present. The degree of complementarity required for hybridization may vary depending on hybridization conditions, and in particular, may be controlled by the binding temperature.

The term "target gene" as used herein means a nucleic acid sequence (base sequence) to be activated, inhibited or labeled, and there is no difference from the term "target nucleic acid", and these two terms are used interchangeably in the present specification.

In the present invention, when the target nucleic acid (base sequence) containing the target gene is brought into contact with (bound to) the complex *in vitro* or *in vivo,* the bioactive nucleic acid is released from the carrier peptide nucleic acid and thus becomes biologically active.

In the present invention, the diseases that can be prevented and treated using the nucleic acid complex may be determined depending on the target gene to which the bioactive nucleic acid in the nucleic acid complex binds. In the present invention, the target gene to which the bioactive nucleic acid binds is TLR2 or IL-4R$\alpha$.

Therefore, in the present invention, diseases that can be prevented and treated using the nucleic acid complex are preferably used for the treatment of skin diseases, for example, psoriasis, atopic diseases including atopic dermatitis, and skin cancer such as melanoma, keloid diseases, diseases such as skin damage and pigmentation, tumors or cancers, inflammatory diseases, senile macular degeneration, diabetic retinopathy, rare and severe diseases, cardiovascular diseases, metabolic diseases, etc., but are not limited thereto.

Meanwhile, in the present invention, the term "therapeutic composition" may be used interchangeably with "pharmaceutical composition", and indicates a composition that contains, as an active ingredient, a nucleic acid complex containing the bioactive nucleic acid and the carrier peptide nucleic acid bound to the nucleic acid according to the present invention, and may further contain a therapeutic drug for treating a desired disease, bound to the nucleic acid complex.

The therapeutic composition of the present invention may be formulated in a form deliverable through the skin according to standard pharmaceutical practice. In addition to the active ingredient, these formulations may contain additives such as carriers, excipients, adjuvants or diluents suitable for formulations in a form that is pharmaceutically acceptable, particularly applicable to the skin.

Preferably, the composition according to the present invention may be formulated in the form of an aqueous solution, gel, ointment, cream, lotion, paste, smear or patch.

Most preferably, the composition may be formulated in the form of an aqueous solution, and the aqueous solution may be in the form of distilled water and a buffer solution.

The term "physiologically acceptable" refers to a property that does not impair the biological activity and physical properties of a compound.

The term "carrier" is defined as a compound that facilitates the transport of the nucleic acid complex into cells or tissues. For example, dimethylsulfoxide (DMSO) is a commonly used carrier that facilitates the incorporation of many organic compounds into cells or tissues of an organism.

The term "diluent" is defined as a compound that stabilizes a biologically active form of a target compound and is diluted in water that dissolves the target compound. Salts dissolved in buffer solutions are used as diluents in the art. A commonly used buffer solution is phosphate-buffered saline because it mimics the salinity of human bodily fluids. Because buffer salts can control the pH of a solution at low concentrations, buffer diluents rarely alter the biological activity of compounds.

The substance containing the nucleic acid complex in the present invention may be administered to a patient alone or as a pharmaceutical composition mixed with other active ingredients, or with suitable carriers or excipients, that is, in combination therapy.

The pharmaceutical composition suitable for use in the present invention include compositions containing the active ingredients in an amount effective to achieve the intended purpose thereof. More specifically, a therapeutically effective amount means an amount of a compound effective to lengthen the survival of the subject to be treated, or to prevent, alleviate or relieve the symptoms of diseases. The determination of the therapeutically effective amount is possible by one those skilled in the art, particularly in consideration of the detailed description provided herein.

The term "prevention" as used herein means any action of preventing the onset of a disease or inhibiting the progression thereof by administration (or application) of the therapeutic composition containing the skin-permeable nucleic acid complex.

The term "alleviation" as used herein refers to any action of at least reducing the degree of parameters related to the condition to be treated, for example, the severity of symptoms, by administration (or application) of the therapeutic composition containing the skin-permeable nucleic acid complex.

In addition, the term "treatment" as used herein refers to any action in which symptoms of a disease are alleviated or eliminated by administration (or application) of the therapeutic composition containing the skin-permeable nucleic acid complex.

In the present invention, the skin-permeable nucleic acid complex may be administered (or applied) using a carrier such as a liposome. The liposome may enable the complex to target specific tissues such as lymphatic tissue, or to selectively target infected cells, and may also facilitate the increase in the half-life of the composition containing the complex. Liposomes include emulsions, foams, micelles, insoluble monolayers, liquid crystals, phospholipid dispersions, lamellar layers and the like. In such formulations, the complex to be delivered may be incorporated, as a part of liposome, alone or in combination with a molecule that binds to a predominant receptor, among certain cells, lymphocytes, such as monoclonal antibodies that bind to CD45 antigens, or in combination with other therapeutic compositions. Thus, liposomes filled or decorated with the predetermined complex of the present invention that deliver the nucleic acid complex composition may be directed to the site of the lymphocytes.

Liposomes for use in accordance with the present invention are generally formed from standard vesicleforming lipids including neutral and negatively charged phospholipids and sterols such as cholesterol. In general, lipids are selected in consideration of, for example, stability of liposomes in the bloodstream, acid lability, and size of liposomes. Various methods may be used for the preparation of liposomes. For example, the method described in the following documents [Szoka, et al., Ann. Rev. Biophys. Bioeng., 9:467, 1980), and US Patent Nos. 4,235,871, 4,501,728, 4,837,028 and 5,019,369] may be used.

In another aspect, the present invention is directed to a method of preventing or treating skin diseases including administering, to a subject, a skin-permeable nucleic acid complex containing a bioactive nucleic acid having a sequence capable of binding to TLR2 or IL-4Rα and a carrier peptide nucleic acid, which are complementarily bound to each other.

The composition containing the nucleic acid complex according to the present invention may be applied to the skin in a pharmaceutically effective amount to treat skin diseases or to inhibit (or alleviate) the symptoms of skin diseases. The pharmaceutically effective amount may vary depending on various factors such as the type of skin disease, the age and weight of the patient, the characteristics and extent of symptoms, the type of current therapy, the number of treatments that are performed, and the application form and route, and can be easily determined by experts in the field. The composition of the present invention may be applied simultaneously or sequentially in combination with the pharmacological or physiological components described above, and may be applied sequentially or simultaneously in combination with additional conventional therapeutic agents. Administration may be performed in one or multiple applications.

As used herein, the term "subject" refers to a mammal, preferably a human, that suffers from or is at risk of a condition or disease that can be alleviated, suppressed or treated by administering (applying) the skin-permeable nucleic acid complex according to the present invention thereto.

In addition, the amount of the compound of the present invention that is administered (applied) to the human body may vary depending on the age, weight and gender of the patient, the administration (application) form, the health status, and the severity of disease, and is generally 0.001 to 1,000 mg/day, preferably 0.01 to 500 mg/day, based on an adult patient weighing 70 kg, and may be administered (applied) once a day or in multiple doses (in a portionwise manner) several times a day at regular time intervals according to the prescription of doctors or pharmacists.

The toxicity and therapeutic efficiency of the compositions containing the skin-permeable nucleic acid complex described herein are, for example, estimated through standard pharmaceutical procedures in cell culture or laboratory animals to determine the LD50 (lethal dose for 50% of the population), ED50 (dose providing a therapeutic effect on 50% of the population) and IC50 (dose providing therapeutic and inhibitory effects on 50% of the population). The ratio of toxicity to therapeutic effect for a dose is called the therapeutic index, and may be expressed as the ratio of LD50 to ED50 (or IC50). Compounds having a high therapeutic index are preferred. The data obtained from these cell culture assays may be used to estimate the range of dose for human applications. The amount (dosage) of such a compound that is administered or applied is preferably within a range of a circulating concentration including ED50 (or IC50) with little or no toxicity.

As used herein, the term "administration" refers to an act of introducing the pharmaceutical composition of the present invention into a subject by any suitable method, and the administration may be performed through any of various routes, either oral or parenteral, as long as it enables the composition to reach the target tissue.

The pharmaceutical composition of the present invention may be administered through any general route that enables the composition to reach the target tissue. The pharmaceutical composition of the present invention may be administered intraperitoneally, intravenously, intramuscularly, subcutaneously, intradermally, orally, intranasally, pulmonarily or rectally as desired, but is not limited thereto. In addition, the composition may be administered by any device capable of delivering the active substance to the target cells.

The pharmaceutical composition of the present invention may be administered in a pharmaceutically effective amount. The term "pharmaceutically effective amount" used herein means a sufficient amount used to treat or prevent a disease at a reasonable benefit/risk ratio applicable to medical treatment or prevention. The effective amount is determined depending on factors including the severity of the disease, the activity of the drug, the age, weight, health and gender of the patient, the sensitivity of the patient to the drug, the time of administration, the route of administration, and the rate of excretion and treatment period of the composition of the present invention used, drugs used in combination with or concurrently with the composition of the present invention, and other factors well known in the pharmaceutical field.

The pharmaceutical composition of the present invention may be administered as a single therapeutic agent or in combination with other therapeutic agents, either sequentially or simultaneously. The pharmaceutical composition of the present invention may be administered in single or multiple doses. Taking into consideration these factors, it is important to administer the composition in the minimum amount sufficient to achieve maximum efficacy without side effects.

In addition, the dosage (administered amount) of the pharmaceutical composition according to the present invention may be determined by those skilled in the art in consideration of the purpose of use, the severity of the disease, the patient's age, weight, gender, and history, or the substances used as active ingredients. For example, the pharmaceutical composition may be administered to an adult in a daily dose of 10 mg/kg to 100 mg/kg, more preferably 10 mg/kg to 30 mg/kg. The frequency of administration of the composition of the present invention is not particularly limited, and the composition may be administered one to three times a day, or may be divided into multiple doses and administered throughout the day.

In another aspect, the present invention is directed to the use of a skin-permeable nucleic acid complex containing a bioactive nucleic acid having a sequence capable of binding to TLR2 or IL-4R$\alpha$ and a carrier peptide nucleic acid, which are complementarily bound to each other for the prevention or treatment of skin diseases.

In another aspect, the present invention is directed to the use of a skin-permeable nucleic acid complex containing a bioactive nucleic acid having a sequence capable of binding to TLR2 or IL-4R$\alpha$ and a carrier peptide nucleic acid, which are complementarily bound to each other for the preparation of a drug for preventing or treating skin diseases.

The cosmetic composition of the present invention can be used in any formulation to be applied to the skin. More specifically, the cosmetic composition may be prepared in a formulation selected from cosmetic products such as a skin lotion, skin softener, skin toner, astringent, lotion, milk lotion, moisturizing lotion, nutrition lotion, massage cream, nutrition cream, moisture cream, hand cream, foundation, essence, nutrition essence, spray and pack, as well as a soap, cleansing foam, cleansing lotion, cleansing cream, body lotion, body cream, body oil, body cleaner, shampoo, ointment, patch (hydrogel slimming patch), and the like, but is not limited thereto. In addition, the cosmetic composition may be prepared as a skin-contacting material, such as a cosmetic, detergent or fiber, that comes into contact with the skin.

The cosmetic composition of the present invention may be appropriately selected depending on the purpose.

When the formulation of the present invention is a paste, cream or gel, a carrier component thereof may be animal oil, vegetable oil, wax, paraffin, starch, tragacanth, a cellulose derivative, polyethylene glycol, silicone, bentonite, silica, talc, zinc oxide, or the like.

When the formulation of the present invention is a powder or spray, a carrier component thereof may be a lactose, talc, silica, aluminum hydroxide, calcium silicate or polyamide powder. In particular, when the formulation of the present invention is a spray, it may further contain a propellant such as chlorofluorohydrocarbon, propane/butane, or dimethyl ether.

When the formulation of the present invention is a solution or emulsion, a carrier component thereof may be a solvent, a solubilizing agent or an emulsifying agent. Examples of the carrier component include water, ethanol, isopropanol, ethyl carbonate, ethyl acetate, benzyl alcohol, benzyl benzoate, propylene glycol, 1,3-butylglycol oil, glycerol aliphatic ester, polyethylene glycol and fatty acid ester of sorbitan.

When the formulation of the present invention is a suspension, a carrier component thereof may be a liquid diluent such as water, ethanol or propylene glycol, a suspending agent such as ethoxylated isostearyl alcohol, polyoxyethylene sorbitol ester or polyoxyethylene sorbitan ester, microcrystalline cellulose, aluminum metahydroxide, bentonite, agar or tragacanth, or the like.

When the formulation of the present invention is a surfactant-containing cleanser, a carrier component thereof may be aliphatic alcohol sulfate, aliphatic alcohol ether sulfate, sulfosuccinic acid monoester, isethionate, an imidazolinium derivative, methyltaurate, sarcosinate, fatty acid amide ether sulfate, alkylamidobetaine, aliphatic alcohol, fatty acid glyceride, fatty diethanolamide, vegetable oil, lanolin derivative, or ethoxylated glycerol fatty acid ester.

In addition, the external preparation for skin of the present invention may be prepared by mixing, in addition to the components described above, predetermined amounts of various known ingredients mixed with cosmetic ingredients applied to the skin or mucous membranes, or pharmaceuticals or quasi-drugs for external use.

Specifically, the cosmetic composition of the present invention may be prepared by mixing with other ingredients that are usually blended in cosmetics as needed in each formulation. The blending ingredients that may be added include: antioxidants (e.g., carboxylic acids such as ascorbic acid and citric acid; or phenols such as tocopherol and dibutyl hydroxytoluene), humectants (e.g., glycols such as glycerin, propylene glycol, dipropylene glycol, and 1,3-butylene glycol; organic salts such as hyaluronic acid; amides such as urea), thickeners (for example, polymer compounds such as polyethylene glycol and; cellulose such as sodium carboxymethyl cellulose and carboxypropyl cellulose), buffers (e.g., organic acids such as citric acid, lactic acid and tartaric acid; inorganic acids such as hydrochloric acid and boric acid; salts such as sodium dihydrogen phosphate and sodium citrate; organic bases such as triethanolamine; and inorganic bases such as sodium hydroxide and potassium hydroxide), adsorbents (e.g., hydrous aluminum silicates such as kaolin and bentonite; and inorganic salts such as magnesium alumina hydroxide and aluminum hydroxide), a base (e.g., organic substances such as white petrolatum, Tween60, Tween80, liquid paraffin, beeswax, Vaseline, castor oil, silicone oil, hydrogenated castor oil, natural rubber, palm oil fatty acid diethanolamide, polyoxyethylene hydrogenated castor oil, natural rubber latex, and 1,3-pentadiene copolymer resin; polymer compounds such as polybutene, synthetic rubber (SBR), polyethylene glycol monostearate, polyoxyethylene glycol monostearate, polyoxyethylene cetstearyl ether, polyoxyethylene oleylcetyl ether, silicone, starch acrylate 300, sodium polyacrylate, n-butyl methacrylate·acrylate copolymers and carboxyvinyl polymers; fatty acids such as stearic acid; alcohols such as cetanol and myristyl alcohol; fatty acid esters such as octadodecyl myristate, isopropyl myristate, and cetyl octanoate, solvents (e.g., ethanol, isopropanol, 1,3-butylene glycol, n-octadecyl alcohol, crotamiton, and carbohydrates such as tri(caprylic·capric)glycerin), stabilizers (e.g. inorganic salts such as sodium metaphosphate, zinc oxide and titanium oxide; organic salts such as sodium polyoxyethylene lauryl sulfate ether sulfate and sodium lauryl sulfate), adhesives (e.g., polymer compounds such as sodium polyacrylate and dipropylene glycol), emulsifiers (e.g., carbohydrates such as sorbitan monooleate, polyoxyethylene sorbitan monooleate, D-sorbitol, polyglycerin monolaurate and sodium polyoxyethylene lauryl ether sulfate), surfactants (e.g., polymer compounds such as polyglycerin monolaurate and polyoxyethylene oleyl alcohol ether), fragrances, colors (dyes, pigments), metal blockers, deodorants, coatings, ultraviolet absorbers, ultraviolet scatters, vitamins, and the like.

The cosmetic composition of the present invention may be applied in an appropriate amount to the skin depending on the skin area in need of application, and may be used repeatedly once to several times a day as necessary. The amount to be applied and the number of applications may be appropriately increased or decreased as necessary depending on the skin condition of the subject, the formulation, the age, gender, weight, and response sensitivities of the subject to be administered, the application period, and the like.

Those skilled in the art can appropriately select a dosage effective for the desired effect. The formulation of the composition may be a single-dose or multiple-dose formulation, and the daily effective amount may be optionally administered in multiple doses several times.

The pharmaceutical or cosmetic composition of the present invention may be applied to the skin by a method such as iontophoresis, sonophoresis, electroporation, microelectric patch, mechanical pressure, osmotic pressure gradient, occlusive cure or microinjection therapy, or a combination thereof in order to improve the permeability of the active ingredient.

## Example

Hereinafter, the present invention will be described in more detail with reference to the following examples. However, it will be obvious to those skilled in the art that the following examples are provided only for illustration of the present invention and should not be construed as limiting the scope of the present invention based on the subject matter of the present invention.

### Example 1: Bioactive nucleic acid and carrier peptide nucleic acid, and preparation of complex using the same

In order to verify the effect on atopic dermatitis of the nucleic acid complex of the present invention, atopic dermatitis-targeting genes, namely, TLR2 (Toll-like receptor 2) and IL-4Rα (interleukin-14 receptor α) were used as target genes. TLR2, a gene that is expressed when allergens or bacteria penetrate into the skin, is overexpressed in atopic dermatitis patients, thus worsening atopic dermatitis due to increased inflammation by inflammatory cytokines in the skin. For this reason, TLR2 is predicted to be an important target in atopic dermatitis.

Mutation of IL-4Rα, which is a mutation characteristic of atopic dermatitis patients, is known to be a factor that induces atopic dermatitis by disrupting the homeostasis of differentiation into T helper 1/2 type in the differentiation of T cells.

In order to determine the therapeutic effect on atopic dermatitis, an antisense peptide nucleic acid (antisense PNA) was used as the bioactive nucleic acid for TLR2 and IL-4Rα.

The bioactive nucleic acid (antisense PNA) used as a control of the present invention has a sequence represented by SEQ ID NOS: 1 and 3, and the bioactive nucleic acid (antisense PNA) used to determine the therapeutic effect on atopic dermatitis has a sequence represented by SEQ ID NOS: 2 and 4.

The peptide-nucleic-acid-based bioactive nucleic acid used in this example binds GLFDIIKKIAESF (SEQ ID NO: 19), which is a peptide for facilitating the endosomal escape, to the 5' end thereof, and the nucleotide sequence, monomer modification and structure thereof are shown in Table 1 below. Table 1 below shows sequence information of the bioactive nucleic acid and the carrier peptide nucleic acid used as the control, and sequence information of the bioactive nucleic acid and the carrier peptide nucleic acid used to determine the effect as a therapeutic agent for atopic dermatitis targeting TLR2 and IL-4R. The bioactive nucleic acids of SEQ ID NOS: 1 and 2 and the carrier nucleic acids of SEQ ID NOS: 5 to 13 target TLR2, and the bioactive nucleic acids of SEQ ID NOS: 3 and 4 and the carrier nucleic acids of SEQ ID NOS: 14 to 18 target IL-4Rα.

All of the peptide nucleic acids used in the present invention were synthesized in PANAGENE (Korea) through HPLC purification. Some of all the carrier peptide nucleic acids according to the embodiment of the present invention bind a peptide such as histidine (10) for facilitating endosomal escape to the 5' or 3' end thereof, and have the sequences represented by SEQ ID NOS: 5 to 18 (Table 1).

[Table 1]

| | | Sequences of bioactive nucleic acids and carrier peptide nucleic acids for verification of therapeutic effect on atopic dermatitis | |
|---|---|---|---|
| Item | SEQ ID NO | Base sequence | Monomer modification |
| Bioactive nucleic acid | SEQ ID NO: 1 | 5'-GLFDIIKKIAESF-O-AGT$^{(-)}$GCG$^{(+)}$CAT$^{(-)}$TGG$^{(+)}$TCT$^{(-)}$AT-O-K-3' | -+-+- |
| | SEQ ID NO: 2 | 5'-GLFDIIKKIAESF-O-A$^{(-)}$TGT$^{(+)}$AGG$^{(-)}$TG$^{(+)}$ATCC$^{(-)}$TGTT-O-K-3' | -+-+- |
| | SEQ ID NO: 3 | 5'-GLFDIIKKIAESF-O-AG$^{(-)}$TA$^{(+)}$GTCG$^{(-)}$CGT$^{(+)}$ATG$^{(-)}$T-O-K | -+-+- |
| | SEQ ID NO: 4 | 5'-GLFDIIKKIAESF-O-AAG$^{(-)}$CC$^{(+)}$ACC$^{(-)}$CC$^{(+)}$ATT$^{(-)}$GG-O-K | -+-+- |
| Carrier peptide nucleic acid | SEQ ID NO: 5 | 5'-K-0-AT$^{(+)}$AGACCA$^{(+)}$ATGCGC$^{(+)}$ACT-O-Histidine(10)-3' | +++ |
| | SEQ ID NO: 6 | 5'-K-O-A$^{(+)}$AC$^{(+)}$AG-O-$\alpha$-3' | ++ |
| | SEQ ID NO: 7 | 5'-$\alpha$-O-G$^{(+)}$ AC$^{(+)}$AA-O-K-3' | ++ |
| | SEQ ID NO: 8 | 5'-K-O-A$^{(+)}$AC$^{(+)}$AG-O-Histidine(10)-3' | ++ |
| | SEQ ID NO: 9 | 5'-Histidine(10)-O-G$^{(+)}$AC$^{(+)}$AA-O-K-3' | ++ |
| | SEQ ID NO: 10 | 5'-K-O-A$^{(+)}$ACAGGA$^{(+)}$TCACCT$^{(+)}$ACAT-3' | +++ |
| | SEQ ID NO: 11 | 5'-T$^{(+)}$ACATCC$^{(+)}$ACTAGG$^{(+)}$ACAA-O-K-3' | +++ |
| | SEQ ID NO: 12 | 5'-K-O-A$^{(+)}$ACAGGA$^{(+)}$TCACCT$^{(+)}$ACAT-O-Histidine(10)-3' | +++ |
| | SEQ ID NO: 13 | 5'-Histidine(10)-O-T$^{(+)}$ACATCC$^{(+)}$ACTAGG$^{(+)}$ACAA-O-K-3' | +++ |
| | SEQ ID NO: 14 | 5'-Histidine(10)-O-TC$^{(+)}$ATCAGC$^{(+)}$GCATAC$^{(+)}$A-O-K | +++ |
| | SEQ ID NO: 15 | 5'-K-O-CC$^{(+)}$AA$^{(+)}$T-O-Histidine(10)-3' | ++ |
| | SEQ ID NO: 16 | 5'-Histidine(10)-O-T$^{(+)}$AA$^{(+)}$CC-O-K-3' | ++ |
| | SEQ ID NO: 17 | 5'-K-O-CC$^{(+)}$AATGG$^{(+)}$GGTGG$^{(+)}$CTT-O-Histidine(10)-3' | +++ |
| | SEQ ID NO: 18 | 5'-Histidine(10)-O-TTC$^{(+)}$GGTGG$^{(+)}$GGTAA$^{(+)}$CC-O-K-3' | +++ |

In order to impart electrical properties, modification of the monomer is designed such that the peptide nucleic acid backbone contains lysine (Lys, K$^{(+)}$) for a positive electrical charge and the modified peptide nucleic acid backbone contains glutamic acid (Glu, E$^{(-)}$) for a negative electrical charge.

The combination of respective bioactive nucleic acids and carrier peptide nucleic acids was hybridized in the presence of DMSO to produce a complex containing the bioactive nucleic acid and the carrier peptide nucleic acid.

**Example 2: Analysis of therapeutic effect in atopic-dermatitis-like cell model** using **skin-permeable nucleic acid complex**

The therapeutic effect on atopic dermatitis was analyzed using the skin-permeable nucleic acid complex containing the bioactive peptide nucleic acid and the carrier peptide nucleic acid using TLR2 as a target gene, prepared to have the structure of the following Table 2 according to Example 1.

[Table 2]

| Sequences of bioactive nucleic acids and carrier peptide nucleic acids for inhibition of TLR2 activity | | | |
|---|---|---|---|
| Item | SEQ ID NO | Base sequence | Monomer modification |
| Bioactive nucleic acid | SEQ ID NO: 2 | 5'-GLFDIIKKIAESF-O-A$^{(-)}$TGT$^{(+)}$AGG$^{(-)}$TG$^{(+)}$ATCC$^{(-)}$TGTT-O-K-3' | -+-+- |
| Carrier peptide nucleic acid | SEQ ID NO: 6 | 5'-K-O-A $^{(+)}$AC$^{(+)}$AG-O-$\alpha$-3' | ++ |
| | SEQ ID NO: 7 | 5'-$\alpha$-O-G$^{(+)}$AC$^{(+)}$AA-O-K-3' | ++ |
| | SEQ ID NO: 8 | 5'-K-O-A$^{(+)}$AC$^{(+)}$AG-O-Histidine(10)-3' | ++ |
| | SEQ ID NO: 9 | 5'-Histidine(10)-O-G$^{(+)}$AC$^{(+)}$AA-O-K-3' | ++ |
| | SEQ ID NO: 10 | 5'-K-O-A$^{(+)}$ACAGGA$^{(+)}$TCACCT$^{(+)}$ACAT-3' | +++ |
| | SEQ ID NO: 11 | 5'-T$^{(+)}$ACATCC$^{(+)}$ACTAGG$^{(+)}$ACAA-O-K-3' | +++ |
| | SEQ ID NO: 12 | 5'-K-O-A$^{(+)}$ACAGGA$^{(+)}$TCACCT$^{(+)}$ACAT-O-Histidine(10)-3' | +++ |
| | SEQ ID NO: 13 | 5'-Histidine(10)-O-T$^{(+)}$ACATCC$^{(+)}$ACTAGG$^{(+)}$ACAA-O-K-3' | +++ |

Example 2-1: Cell culture

Human keratinocytes (HaCaT) obtained from CLS (CLS Cell Lines Service, Germany) were incubated at 37°C in the presence of 5% (v/v) $CO_2$ in DMEM culture medium (Dulbecco's Modified Eagle's Medium, Wellgene, Korea) supplemented with 10% (v/v) fetal bovine serum, 100 units/ml of penicillin, and 100 µg/ml of streptomycin. To construct an atopic-dermatitis-like cell model, the cells were treated with 5 ng/mL of a house dust mite extract and 5 $\mu$M of DNCB (2-dinitrochlorobenzene) and incubated for 24 hours.

Example 2-2: Analysis of cell viability in keratin cell lines using MTT assay

A human-derived keratin cell line was seeded at a density of $6 \times 10^3$ cells/well in a 96-well plate under the same experimental conditions as in Example 2-1, incubated for 24 hours, and then treated with the complex containing a bioactive nucleic acid and a carrier peptide nucleic acid, prepared to have the structure of Table 2. The resulting cell line was treated at 20 $\mu$L/well with 5 mg/mL of a MTT (3-(4,5-dimethylthiazol-2-yl)-2,5-diphenyltetrazolium bromide) solution in 1X PBS for 4 hours. After incubation, OD (optical density) was measured and analyzed using a spectrophotometer.

The nucleic acid complex combinations that were used in this example are shown in Table 3 below.

[Table 3]

| Combination of nucleic acid complexes for cell viability analysis in keratin cell lines | |
|---|---|
| Item | Nucleic acid complex |
| 1 | SEQ ID NOS: 2 and 6 |
| 2 | SEQ ID NOS: 2 and 7 |
| 3 | SEQ ID NOS: 2 and 8 |
| 4 | SEQ ID NOS: 2 and 9 |
| 5 | SEQ ID NOS: 2 and 10 |
| 6 | SEQ ID NOS: 2 and 11 |
| 7 | SEQ ID NOS: 2 and 12 |
| 8 | SEQ ID NOS: 2 and 13 |

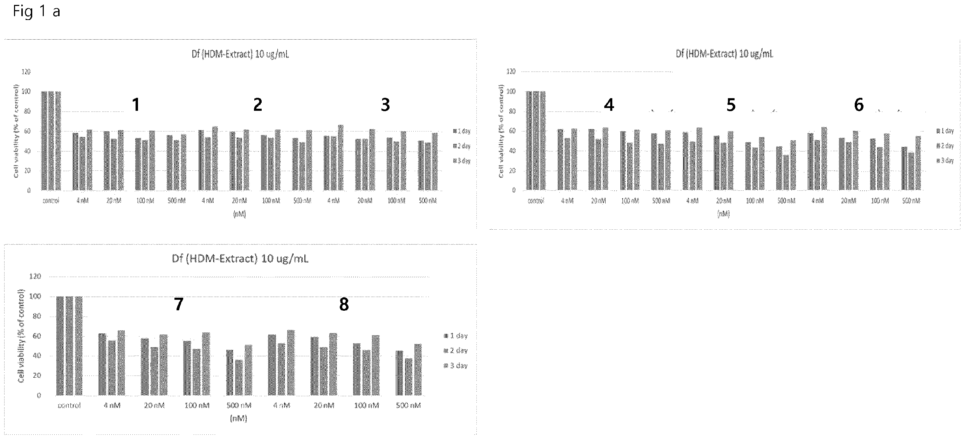

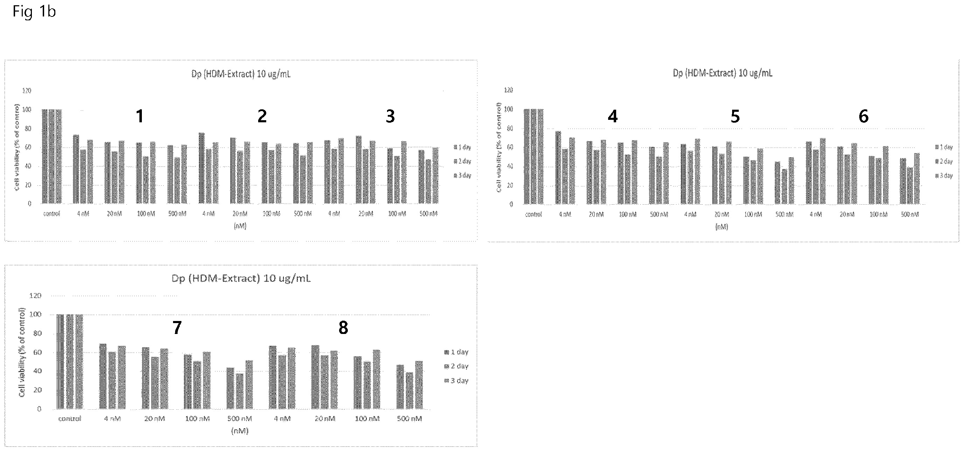

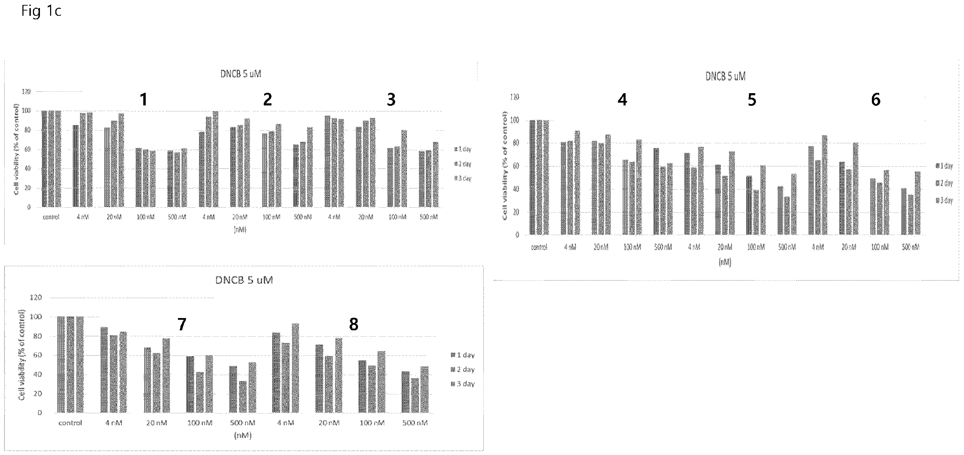

As a result, as can be seen from FIGS. 1A to 1C, the cell viability was decreased in a concentration-dependent manner due to the nucleic acid complex in the atopic-dermatitis-like cell model induced using house dust mite extract or DNCB.

Example 2-3: Analysis of gene expression using Western blot assay

The human-derived keratin cell line was seeded at a density of $1 \times 10^5$ cells/well in a 6-well plate under the same conditions as in Example 2-1, incubated for 24 hours, treated with the complex containing a bioactive peptide nucleic acid and a carrier peptide nucleic acid, and incubated for 24, 48 and 72 hours. 30 $\mu$L of RIPA buffer was added to each well to obtain a protein lysate. The proteins in the protein lysate were assayed using a BCA assay kit (Thermo Fisher, USA), and 30 $\mu$g of protein was separated according to size using electrophoresis. After transfer to the PVDF membrane, the protein was treated at 1:1000 with TLR2 (Santa Cruz Biotech., USA), p-NFkB (Cell Signaling Technology, USA), MyD88 (Cell Signaling Technology, USA), and TARC (Abcam, USA) as primary antibodies and was allowed to stand at 4°C for one day. The result was washed using 1X TBS-T, treated at 1:2000 with goat anti-rabbit and goat anti-mouse secondary antibodies (Santa Cruz Biotech., USA) and allowed to stand at room temperature for 2 hours. The result was treated with Supersignal™ West Femto Maximum Sensitivity Substrate (Thermo Fisher, USA) and the efficiency of suppression of expression of target genes in keratin cell lines was analyzed using an Image600 (Amersham, Germany) apparatus.

In this example, nucleic acid combinations that were found to have effects through Example 2-2 were selected, and changes in TLR2 and downstream gene expression in an atopic-dermatitis-like cell model using house dust mites and sick-house-syndrome-causing chemicals were analyzed. The nucleic acid combinations are shown in Table 4 below.

[Table 4]

| Combination of nucleic acid complexes for TLR2 and downstream gene expression analysis in keratinocyte lines | |
|---|---|
| Item | Nucleic acid complex |
| 1 | SEQ ID NOS: 2 and 8 |

(continued)

| Combination of nucleic acid complexes for TLR2 and downstream gene expression analysis in keratinocyte lines | |
|---|---|
| Item | Nucleic acid complex |
| 2 | SEQ ID NOS: 2 and 6 |
| 3 | SEQ ID NOS: 2 and 10 |
| 4 | SEQ ID NOS: 2 and 7 |
| 5 | SEQ ID NOS: 2 and 5 |
| 6 | SEQ ID NOS: 2 and 9 |

For the combination of nucleic acid complexes in Table 4, expression patterns of TLR2 proteins and downstream genes in an atopic-dermatitis-like cell model were analyzed.

As a result, as can be seen from FIGS. 2A to 2C, the expression of the target gene and the expression of downstream gene were inhibited most over time in a concentration-dependent manner by the nucleic acid complex combinations of SEQ ID NOS: 2 to 7.

**Example 3: Analysis of therapeutic effect in atopic dermatitis animal model using skin-permeable nucleic acid complex**

Example 3-1: Analysis of phenotypic effect of atopic dermatitis in atopic-dermatitis-induced animal model using house dust mite extract

The backs of NC/Nga mice were depilated, and 100 mg of AD cream (house dust mite extract cream, Biostir, Japan) was applied thereto twice a week over a total of 3 weeks to construct an animal model induced with atopic dermatitis using house dust mites. The animal model was treated with the cream-type nucleic acid complex a total of twice a week, the phenotype of the atopic dermatitis animal model was imaged, and the degree of hair growth on the back was measured with ImageJ.

In this Example, the nucleic acid combination found to have an effect through Example 2-2 was selected, and phenotypic and histological findings and changes in TLR2 and downstream gene expression were analyzed in the atopic-dermatitis-like animal model using the house dust mite extract. The nucleic acid complex combination is shown in Table 5 below.

[Table 5]

| Combination of nucleic acid complexes for analysis of therapeutic effects in atopic dermatitis animal model | |
|---|---|
| Item | Nucleic acid complex |
| PNA | SEQ ID NOS: 2 and 12 |

The result showed that the atopic dermatitis phenotype was decreased in the nucleic acid complex group (FIGS. 3A), and that hair loss due to skin damage was reduced in the group to which the nucleic acid complex was applied, compared to the animal group induced with atopic dermatitis (In FIG. 3A, "Cream control" is a group containing a cream formulation but not a nucleic acid complex, and "Positive control" is a cream formulation containing dexamethasone, a conventional therapeutic agent).

Example 3-2: Analysis of changes in IgE and TARC concentrations in serum

In the animal experiment conducted under the same conditions as in Example 3-1, mouse blood was collected through orbital blood collection on the final day and allowed to stand at room temperature for 2 hours or longer, and serum was collected using a centrifuge (14,000 rpm, 15 min). The collected serum was measured to determine the concentrations of IgE and TARC in the serum using experimental methods provided by IgE ELISA kit (Koma Biotech, Korea) and TARC ELISA kit (R&D system, USA) .

As a result, as can be seen from FIGS. 3B and 3C, the concentrations of IgE and TARC decreased to levels similar to that of the negative control group in the group treated with the nucleic acid complex, compared to the control group induced with atopic dermatitis.

Example 3-3: Analysis of gene expression using Western blot assay

The experiment was conducted under the same conditions as in Example 3-1 to obtain a part of the back tissue of biopsied mice, and 200 μL of RIPA buffer was added to each well to obtain a protein lysate. The proteins in the protein lysate were assayed using a BCA assay kit (Thermo Fisher, USA), and 30 μg of protein was separated according to size using electrophoresis. After transfer to the PVDF membrane, the protein was treated at 1:1000 with TLR2 (Santa Cruz Biotech., USA), p-NFkB (Cell Signaling Technology, USA), and MyD88 (Cell Signaling Technology, USA) as primary antibodies, and was allowed to stand at 4°C for one day. The result was washed using 1X TBS-T, treated at 1:2000 with the goat anti-rabbit and goat anti-mouse secondary antibodies (Santa Cruz Biotech., USA) and allowed to stand at room temperature for 2 hours. The result was treated with Supersignal™ West Femto Maximum Sensitivity Substrate (Thermo Fisher, USA) and the efficiency of suppression of expression of target genes in keratin cell lines was analyzed using an Image600 (Amersham, Germany) apparatus.

As can be seen from FIG. 3D, the expression of TLR2 and expression of downstream gene were decreased in the group of the atopic-dermatitis-like animal model tissue treated with the nucleic acid complex.

Example 3-4: Phenotypic analysis in atopic-dermatitis-induced animal model using H&E staining

The experiment was conducted under the same conditions as in Example 3-1, mouse back tissues were biopsied on the final day of the experiment and fixed in 4% formalin solution for one day, and the fixed tissue was embedded in paraffin, sectioned to 5 μm, and mounted on a slide glass. The mounted tissue was stained with a Hematoxylin:Eosin staining solution for a certain period of time, washed with 1X PBS, and analyzed under a microscope.

As a result, as can be seen from FIG. 3E, the thickness of the transdermal tissue and the number of inflammatory cells decreased in the group treated with the nucleic acid complex, compared to the control group induced with atopic dermatitis.

Example 3-5: Analysis of inflammatory markers in tissue in animal model induced with atopic dermatitis using immunostaining

The experiment was conducted under the same conditions as in Example 3-1, mouse back tissues were biopsied at the final day of the experiment and fixed in 4% formalin solution for one day, and the fixed tissue was embedded in paraffin, sectioned to 5 μm, and mounted on a slide glass. The mounted tissue was blocked in 0.5% BSA solution for 1 hour and treated with CD3 and CD11c primary antibody solutions for one day. Subsequently, the primary antibody solution was removed, washed with 1X PBS, treated with a secondary antibody solution, allowed to stand at room temperature for 2 hours, and analyzed by DAB staining.

As a result, as can be seen from FIGS. 3F to 3G, CD3 and CD11c, which are inflammatory markers in tissue, decreased in the nucleic acid complex group, compared to the control group induced with atopic dermatitis, and the numerical values were also observed to be lower when compared (FIG. 3H).

**Example 4: Analysis of therapeutic effect on atopic dermatitis using skin-permeable nucleic acid complex**

The therapeutic effect on atopic dermatitis was analyzed using the skin-permeable nucleic acid complex containing the bioactive peptide nucleic acid and the carrier peptide nucleic acid having IL-4Rα as a target gene, prepared to have the structure of the following Table 6 according to Example 1.

[Table 6]

| Sequences of bioactive nucleic acid and carrier peptide nucleic acids for inhibition of IL-4Rα activity | | | |
|---|---|---|---|
| Item | SEQ ID NO | Base sequence | Monomer modification |
| Bioactive nucleic acid | SEQ ID NO: 4 | 5'-GLFDIIKKIAESF-O-AAG$^{(-)}$CC$^{(+)}$ACC$^{(-)}$CC$^{(+)}$ATT$^{(-)}$GG-O-K | -+-+- |

(continued)

| Sequences of bioactive nucleic acid and carrier peptide nucleic acids for inhibition of IL-4Rα activity | | | |
|---|---|---|---|
| Item | SEQ ID NO | Base sequence | Monomer modification |
| Carrier peptide nucleic acid | SEQ ID NO: 15 | 5'-K-O-CC$^{(+)}$AA$^{(+)}$T-O-Histidine(10)-3' | ++ |
| | SEQ ID NO: 16 | 5'-Histidine(10)-O-T$^{(+)}$AA$^{(+)}$CC-O-K-3' | ++ |
| | SEQ ID NO: 17 | 5'-K-O-CC$^{(+)}$AATGG$^{(+)}$GGTGG$^{(+)}$CTT-O-Histidine(10)-3' | +++ |
| | SEQ ID NO: 18 | 5'-Histidine(10)-O-TTC$^{(+)}$GGTGG$^{(+)}$GGTAA$^{(+)}$CC-O-K-3' | +++ |

Example 4-1: Cell culture

Human keratinocytes (HaCaT) obtained from CLS (CLS Cell Lines Service, Germany) were incubated at 37°C in the presence of 5% (v/v) $CO_2$ in DMEM culture medium (Dulbecco's Modified Eagle's Medium, Wellgene, Korea) supplemented with 10% (v/v) fetal bovine serum, 100 units/ml of penicillin, and 100 μg/ml of streptomycin. To construct an atopic-dermatitis-like cell model, the cells were treated with 10 ng/mL of IL-4 and 10 ng/mL of IL-13 and incubated for 24 hours.

Example 4-2: Analysis of cell viability in keratin cell lines using MTT assay

A human-derived keratin cell line was seeded at a density of $6 \times 10^3$ cells/well in a 96-well plate under the experimental conditions in Example 4-1, incubated for 24 hours, and then treated with the complex containing a bioactive nucleic acid and a carrier peptide nucleic acid, prepared to have the structure of Table 5. The resulting cell line was treated with a 5 mg/mL MTT (3-(4,5-dimethylthiazol-2-yl)-2,5-diphenyltetrazolium bromide) solution in 1X PBS in an amount of 20 μL/well, and incubated for 4 hours. OD (optical density) was measured and analyzed with a spectrophotometer.

The nucleic acid complex combination used in this example is shown in Table 7 below.

[Table 7]

| Combination of nucleic acid complexes for cell viability analysis in keratin cell lines | |
|---|---|
| Item | Nucleic acid complex |
| 1 | SEQ ID NOS: 4 and 15 |
| 2 | SEQ ID NOS: 4 and 16 |
| 3 | SEQ ID NOS: 4 and 17 |
| 4 | SEQ ID NOS: 4 and 18 |

As a result, as can be seen from FIGS. 4A to 4B, the cell viability was decreased in a concentration-dependent manner due to the nucleic acid complex in the atopic-dermatitis-like cell model induced by IL-4 or IL-13.

Example 4-3: Analysis of gene expression using Western blot assay

The human-derived keratin cell line was seeded in a density of $1 \times 10^5$ cells/well in a 6-well plate under the same conditions as in Example 4-1, incubated for 24 hours, treated with the complex containing a bioactive peptide nucleic acid and a carrier peptide nucleic acid, and incubated for each of 24, 48 and 72 hours. 30 μL of RIPA buffer was added to each well to obtain a protein lysate. The proteins in the protein lysate were assayed using a BCA assay kit (Thermo Fisher, USA), and 30 μg of protein was separated according to size using electrophoresis. After transfer to the PVDF membrane, the protein was treated at 1:1000 with the primary antibodies, IL-4Rα (Santa Cruz Biotech., USA), p-JAK3 (Cell Signaling Technology, USA), and p-stat6 (Cell Signaling Technology, USA) and was allowed to stand at 4°C for one day. The result was washed using 1X TBS-T, treated at 1:2000 with the goat anti-rabbit and goat anti-mouse secondary antibodies (Santa Cruz Biotech., USA) and allowed to stand at room temperature for 2 hours. The result was treated with Supersignal™ West Femto Maximum Sensitivity Substrate (Thermo Fisher, USA) and the efficiency of suppression of expression of target genes in keratin cell lines was analyzed using an Image600 (Amersham, Germany) apparatus.

Changes in IL-4Rα and downstream gene expression in an atopic-dermatitis-like cell model using IL-4 and IL-13 were analyzed, and the nucleic acid complex combination that was used is shown in Table 8 below.

[Table 8]

| Combination of nucleic acid complexes for cell viability analysis in keratin cell lines | |
| --- | --- |
| Item | Nucleic acid complex |
| 1 | IL-4 10 ng/mL or IL-13 10 ng/mL |
| 2 | SEQ ID NOS: 4 and 18 |
| 3 | SEQ ID NOS: 4 and 17 |
| 4 | SEQ ID NOS: 4 and 16 |
| 5 | SEQ ID NOS: 4 and 15 |

As a result, as can be seen from FIGS. 5A to 5B, the nucleic acid complex combination of SEQ ID NO: 4 with SEQ ID NO: 15 and of SEQ ID NO: 4 with SEQ ID NO: 16 inhibited IL-4Rα and downstream gene expression in the atopic-dermatitis-like cell model.

**Example 5: Analysis of inhibitory effect on differentiation of T helper type 2 of CD4 positive T lymphocytes using skin-permeable nucleic acid complex**

The effect of inhibiting the differentiation of T helper cells was analyzed using the skin-permeable nucleic acid complex containing the bioactive peptide nucleic acid and the carrier peptide nucleic acid using IL-4Rα as a target gene prepared to have the structure of the following Table 5 in accordance with Example 1.

Example 5-1: Cell culture

Naive T cells (Jurkat, CD4+ naive T cells) obtained from ATCC (American Type Culture Collection, USA) were incubated at 37°C in the presence of 5% (v/v) $CO_2$ in RPMI-1640 culture medium (ATCC, USA) supplemented with 10% (v/v) fetal bovine serum, 100 units/ml of penicillin, and 100 μg/ml of streptomycin. In order to promote differentiation into T helper type 2 cells, the cells were treated with 10 ng/mL of IL-4 (FIG. 6A) and 10 ng/mL of IL-4 + PMA (Phorbol-12-myristate-13-acetate) + Ionomycin (FIG. 6B), and incubated for each of 24, 48 and 72 hours.

Example 5-2: Analysis of gene expression using Western blot assay

Naive T cells (Jurkat, CD4+ naive T cell) were seeded at a density of $2 \times 10^5$ cells/well in a 6-well plate under the same conditions as in Example 5-1, incubated for 24 hours, treated with the complex containing a bioactive peptide nucleic acid and a carrier peptide nucleic acid, and incubated for each of 24, 48 and 72 hours. 30 μL of RIPA buffer was added to each well to obtain a protein lysate. The proteins in the protein lysate were assayed using a BCA assay kit (Thermo Fisher, USA), and 30 μg of protein was separated according to size using electrophoresis. After transfer to the PVDF membrane, the protein was treated at 1:1000 with the primary antibodies IL-4Rα (Santa Cruz Biotech., USA) and p-stat6 (Cell Signaling Technology, USA), and was allowed to stand at 4°C for one day. The result was washed using 1X TBS-T, treated at 1:2000 with the goat anti-rabbit and goat anti-mouse secondary antibodies (Santa Cruz Biotech., USA), and allowed to stand at room temperature for 2 hours. The result was treated with Supersignal™ West Femto Maximum Sensitivity Substrate (Thermo Fisher, USA), and the efficiency of suppression of expression of target genes in keratin cell lines was analyzed using an Image600 (Amersham, Germany) apparatus.

Changes in the expression of IL-4Rα and downstream genes in the T helper type2 differentiation model using IL-4 and IL-4 + PMA were analyzed, and the nucleic acid complex combination that was used is the same as in Table 8 below.

As a result, as can be seen from FIGS. 6A to 6B, the combination of the nucleic acid complexes of SEQ ID NO: 4 and SEQ ID NO: 15 most efficiently inhibited the expression of IL-4Rα and downstream genes in the atopic-dermatitis-like cell model.

**Example 6: Analysis of therapeutic effect on atopic dermatitis using skin-permeable nucleic acid complex in animal model induced with atopic dermatitis by 2-dinitrocholrobenzene (DNCB)**

Example 6-1: Analysis of phenotypic effect of atopic dermatitis in animal model induced with atopic dermatitis using DNCB (2-dinitrochlorobenzene)

NC/Nga mice were acclimated for one week and the backs thereof were depilated, and a 1% DNCB solution (acetone: olive oil = 2:1) was applied to induce primary atopic dermatitis. Then, 0.4% DNCB solution was continuously added twice for a week over a total of 4 weeks to construct an atopic-dermatitis-induced animal model. The atopic-dermatitis-induced animal model was treated with a cream-type nucleic acid complex twice a week over 2 weeks, was treated with 0.4% DNCB solution, and was allowed to stand for a predetermined period. The phenotype of the atopic dermatitis animal model was imaged after treatment with the cream-type nucleic acid complex.

In this Example, the nucleic acid complex having found to have effects through Examples 4 and 5 was selected, and phenotypic and histological findings and changes in IL-4Rα target gene expression in an animal model induced with atopic dermatitis using DNCB were analyzed. The combination is shown in Table 9 below.

[Table 9]

| Combination of nucleic acid complexes for analysis of therapeutic effects in atopic dermatitis animal model | |
| --- | --- |
| Item | Nucleic acid complex |
| PNA | SEQ ID NOS: 4 and 16 |

The result showed that the atopic dermatitis phenotype was decreased in the nucleic acid complex group (FIG. 7A).

Example 6-2: Analysis of gene expression using Western blot assay

The experiment was conducted under the same conditions as in Example 6-1 to obtain a part of the back tissue of biopsied mice, and 200 μL of RIPA buffer was added to each well, to obtain a protein lysate. The proteins in the protein lysate were assayed using a BCA assay kit (Thermo Fisher, USA), and 30 μg of the protein was separated according to size using electrophoresis. After transfer to the PVDF membrane, the protein was treated at 1:1000 with the primary antibody IL-4Rα (Santa Cruz Biotech., USA) and was allowed to stand at 4°C for one day. The result was washed using 1X TBS-T, treated at 1:2000 with goat anti-rabbit and goat anti-mouse secondary antibodies (Santa Cruz Biotech., USA), and allowed to stand at room temperature for 2 hours. The result was treated with Supersignal™ West Femto Maximum Sensitivity Substrate (Thermo Fisher, USA), and the efficiency of suppression of expression of target genes in keratin cell lines was analyzed using an Image600 (Amersham, Germany) apparatus.

As can be seen from FIG. 7B, the expression of IL-4Rα decreased in the skin tissue of the group treated with the nucleic acid complex.

Example 6-3: Analysis of changes in concentration of IgE, TARC and IL-4 in serum

In the animal experiment conducted under the same conditions as in Example 6-1, mouse blood was collected through orbital blood collection on the final day of the experiment and allowed to stand at room temperature for 2 hours or longer, and serum was collected using a centrifuge (14,000 rpm, 15 min). The collected serum was measured to determine the concentrations of IgE, TARC and IL-4 in the serum using experimental methods according to the IgE and IL-4 ELISA kits (Koma Biotech, Korea) and the TARC ELISA kit (R&D system, USA).

As a result, as can be seen from FIG. 8A, the concentrations of IgE, TARC and IL-4 decreased to levels similar to that of the negative control group in the group treated with the nucleic acid complex, unlike the control group induced with atopic dermatitis.

Example 6-4: Analysis of effect of ameliorating pruritus and erythema in atopic-dermatitis-induced animal model through animal behavior analysis

In order to determine the effect of ameliorating pruritus, which is a typical symptom occurring upon induction of atopic dermatitis, in the animal experiment conducted under the same conditions as in Example 6-1, behavioral evaluation of mice between groups was performed in the first week after induction of atopic dermatitis, the nucleic acid complex was administered to the mice at the last week, and comparative behavioral evaluation of mice between groups was conducted to determine the effect of improving pruritus. In addition, to determine whether or not erythema symptoms occurring on the skin when atopic dermatitis is induced are ameliorated, the expression of erythema symptoms was observed during the period of induction of atopic dermatitis, and the effect of ameliorating erythema symptoms was observed when the induction of atopic dermatitis was completed and when the nucleic acid complex was administered.

As a result, as can be seen from FIG. 8B, pruritus and erythema symptoms were remarkably ameliorated in the group treated with the nucleic acid complex compared to the control group induced with atopic dermatitis.

Example 6-5: Phenotypic analysis in atopic-dermatitis-induced animal model using H&E staining

The experiment was conducted under the same conditions as in Example 6-1, mouse back tissues were biopsied on the final day of the experiment and fixed in 4% formalin solution for one day, and the fixed tissue was embedded in paraffin, sectioned to 5 $\mu$m, and mounted on a slide glass. The mounted tissue was stained with a Hematoxylin:Eosin staining solution for a certain period of time, washed with 1X PBS, and analyzed under a microscope.

As a result, as can be seen from FIG. 9E, the thickness of the transdermal tissue and the number of inflammatory cells decreased in the group treated with the nucleic acid complex, unlike the control group induced with atopic dermatitis.

Example 6-6: Analysis of inflammatory markers in tissue in animal model induced with atopic dermatitis using immunostaining

The experiment was conducted under the same conditions as in Example 6-1, mouse back tissues were biopsied on the final day of the experiment and fixed in 4% formalin solution for one day, and the fixed tissue was embedded in paraffin, sectioned to 5 $\mu$m, and mounted on a slide glass. The mounted tissue was blocked in 0.5% BSA solution for 1 hour and treated with CD3 and CD11c primary antibody solutions for one day. Subsequently, the primary antibody solution was removed, washed with 1X PBS, treated with a secondary antibody solution, allowed to stand at room temperature for 2 hours, and analyzed by DAB staining.

As a result, as can be seen from FIGS. 9B, CD3 and CD11c, which are inflammatory markers in tissue, decreased in the nucleic acid complex group, compared to the control group induced with atopic dermatitis, and the numerical values thereof were also observed to be lower when compared.

[Industrial Applicability]

The skin-permeable nucleic acid complex containing the bioactive nucleic acid targeting TLR2 or IL-4R$\alpha$ and the carrier peptide nucleic acid, which are complementarily bound to each other, according to the present invention, exhibits high skin permeability and skin retention (persistence) and thus is useful for prevention, amelioration or treatment of skin diseases such as atopic dermatitis.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

[Sequence Free Text]

An electronic file is attached.

## Claims

1.  A pharmaceutical or cosmetic composition for preventing, ameliorating or treating skin diseases comprising, a skin-permeable nucleic acid complex containing a bioactive nucleic acid having a sequence capable of binding to a TLR2 or IL-4Rα gene; and a carrier peptide nucleic acid, which are complementarily bound to said bioactive nucleic acid as an active ingredient.

2.  The pharmaceutical or cosmetic composition according to claim 1, wherein the bioactive nucleic acid is represented by a sequence selected from the group consisting of SEQ ID NOS: 1 to 4.

3.  The pharmaceutical or cosmetic composition according to claim 1, wherein the carrier peptide nucleic acid is represented by a sequence selected from the group consisting of SEQ ID NOS: 5 to 18.

4.  The pharmaceutical or cosmetic composition according to claim 1, wherein the bioactive nucleic acid capable of binding to the TLR2 gene is represented by the sequence of SEQ ID NO: 2.

5.  The pharmaceutical or cosmetic composition according to claim 1, wherein the bioactive nucleic acid capable of binding to the IL-4Rα gene is represented by the sequence of SEQ ID NO: 4.

6.  The pharmaceutical or cosmetic composition according to claim 1, wherein the nucleic acid complex comprises:

    a bioactive nucleic acid represented by the sequence of SEQ ID NO: 2 or 4; and
    a carrier peptide nucleic acid represented by any one sequence selected from the group consisting of SEQ ID NOS: 5 to 18.

7.  The pharmaceutical or cosmetic composition according to claim 1, wherein a binding force (melting temperature, Tm) between the bioactive nucleic acid capable of binding to the TLR2 or IL-4Rα gene and the carrier peptide nucleic acid is lower than a binding force between the bioactive nucleic acid and the TLR2 or IL-4Rα gene targeted by the bioactive nucleic acid.

8.  The pharmaceutical or cosmetic composition according to claim 1, wherein the bioactive nucleic acid or the carrier peptide nucleic acid has a 5'-end or a 3'-end further bound to a substance that facilitates endosome escape.

9.  The pharmaceutical or cosmetic composition according to claim 8, wherein the substance that facilitates endosome escape comprises at least one selected from the group consisting of peptides, lipid nanoparticles, polyplex nanoparticles, polymer nanospheres, inorganic nanoparticles, cationic lipidbased nanoparticles, cationic polymers, and pH-sensitive polymers.

10. The pharmaceutical or cosmetic composition according to claim 9, wherein the peptide is GLFDIIKKIAESF (SEQ ID NO: 19) or Histidine (10).

11. The pharmaceutical or cosmetic composition according to claim 1, wherein an overall charge of the bioactive nucleic acid is negative or neutral.

12. The pharmaceutical or cosmetic composition according to claim 1, wherein the carrier peptide nucleic acid comprises at least one gamma- or alpha-backbonemodified peptide nucleic acid monomer in order for the carrier peptide nucleic acid to have a positive charge overall.

13. The pharmaceutical or cosmetic composition according to claim 12, wherein the gamma- or alphabackbone-modified peptide nucleic acid monomer comprises more monomers containing a positively charged amino acid than monomers containing a negatively charged amino acid in order for the carrier peptide nucleic acid to have a positive charge overall.

14. The pharmaceutical or cosmetic composition according to claim 13, wherein the positively charged amino acid comprises at least one positive charged amino acid selected from the group consisting of lysine (Lys, K), arginine (Arg, R), histidine (His, H), diamino butyric acid(DAB), ornithine (Orn) and amino acid analogs.

15. The pharmaceutical or cosmetic composition according to claim 13, wherein the negatively charged amino acid is glutamic acid (Glu, E), aspartic acid (Asp, D) or an amino acid analog.

16. The pharmaceutical or cosmetic composition according to claim 1, wherein an overall charge of the nucleic acid complex is positive.

17. The pharmaceutical or cosmetic composition according to claim 1, wherein the nucleic acid complex has skin retention and skin permeability.

18. The pharmaceutical or cosmetic composition according to claim 1, wherein the skin disease comprises any one selected from the group consisting of atopic dermatitis, psoriasis, skin cancer, skin damage, pigmentation, and a keloid disease.

19. A formulation comprising the composition according to claim 1.

20. The formulation according to claim 19, wherein the formulation is any one selected from an aqueous solution, gel, ointment, cream, lotion, paste, smear, and patch.

Fig 1 a

Df (HDM-Extract) 10 ug/mL

Df (HDM-Extract) 10 ug/mL

Df (HDM-Extract) 10 ug/mL

Fig 1b

Fig 1c

Fig 2a

Fig 2b

1 day          2 day          3 day

EP 3 878 474 A1

33

Fig 2c

1 day        2 day        3 day

EP 3 878 474 A1

# Fig 3 a

Negative Control     Positive control     Cream control
dexamethasone

Scramble control     PNA     Scramble control     PNA
0.5 nmole     0.5 nmole     1 nmole     1 nmole

NC : Negative control
PC : Positive control
(cream blended dexamethasone)
CC : Cream control
SC : Scramble control
PNA : 0.5 nmole

NC : Negative control
PC : Positive control
(cream blended dexamethasone)
CC : Cream control
SC : Scramble control
PNA : 1 nmole

Fig 3 b

NC : Negative control
PC : Positive control
(cream blended dexamethasone)
CC : Cream control
SC : Scramble control
PNA : 0.5 nmole

NC : Negative control
PC : Positive control
(cream blended dexamethasone)
CC : Cream control
SC : Scramble control
PNA : 1 nmole

Fig 3 c

EP 3 878 474 A1

Fig 3 d

0.5 nmole group

1 nmole group

EP 3 878 474 A1

# Fig 3 e

Negative control

Positive control

Cream control

Scramble control
0.5 nmole

PNA 0.5 nmole

Scramble control
1 nmole

PNA 1 nmole

Fig 3 f

Negative control

Positive control

Cream control

Scramble control
0.5 nmole

PNA 0.5 nmole

Scramble control
1 nmole

PNA 1 nmole

Fig 3 g

Negative control

Positive control

Cream control

Scramble control
0.5 nmole

PNA 0.5 nmole

Scramble control
1 nmole

PNA 1 nmole

Fig 3 h

NC : Negative control
PC : Positive control
CC : Cream control
SC : Scramble control
PNA : 0.5 nmole

NC : Negative control
PC : Positive control
CC : Cream control
SC : Scramble control
PNA : 1 nmole

EP 3 878 474 A1

Fig 4 a

# Fig 4 b

Fig 5 a

IL-4 : 10 ng/mL

IL-4Rα
p-JAK3
p-stat6
Actin

1 day        2 day        3 day

Fig 5 b

IL-13 : 10 ng/mL

IL-4Rα
p-JAK3
p-stat6
Actin

1 day        2 day        3 day

## Fig 6 a

|  | C 1 2 3 4 5 | C 1 2 3 4 5 | C 1 2 3 4 5 |
| --- | --- | --- | --- |
| IL-4Rα | | | |
| p-stat6 | | | |
| Actin | | | IL-4 : 10 ng/mL |
| | 1 day | 2 day | 3 day |

## Fig 6 b

|  | C 1 2 3 4 5 | C 1 2 3 4 5 | C 1 2 3 4 5 |
| --- | --- | --- | --- |
| IL-4Rα | | | |
| p-stat6 | | | |
| Actin | | | IL-4 + PMA + Ionomycin |
| | 1 day | 2 day | 3 day |

EP 3 878 474 A1

Fig 7a

**Skin Biopsy**

Fig 7b

Fig 8a

Fig 8b

Fig 9a

H&E

Fig 9b

CD3

CD11c

## INTERNATIONAL SEARCH REPORT

| International application No. |
|---|
| **PCT/KR2019/013970** |

| A. CLASSIFICATION OF SUBJECT MATTER |
|---|
| *A61K 48/00(2006.01)i, A61K 47/54(2017.01)i, A61K 31/7088(2006.01)i, A61K 9/00(2006.01)i, A61P 17/00(2006.01)i, A61K 8/60(2006.01)i, A61Q 19/00(2006.01)i* |
| According to International Patent Classification (IPC) or to both national classification and IPC |

| B. FIELDS SEARCHED |
|---|
| Minimum documentation searched (classification system followed by classification symbols) |
| A61K 48/00; A61K 38/10; A61K 47/54; C07K 7/08; C12N 15/09; C12Q 1/68; C12Q 1/6883; A61K 31/7088; A61K 9/00; A61P 17/00; A61K 8/60; A61Q 19/00 |
| Documentation searched other than minimum documentation to the extent that such documents are included in the fields searched<br>Korean utility models and applications for utility models: IPC as above<br>Japanese utility models and applications for utility models: IPC as above |
| Electronic data base consulted during the international search (name of data base and, where practicable, search terms used)<br>eKOMPASS (KIPO internal) & Keywords: TLR2, IL-4Ra, atopy, bioactive nucleic acid, carrier peptide nucleic acid, endosome escape |

| C. DOCUMENTS CONSIDERED TO BE RELEVANT |
|---|

| Category* | Citation of document, with indication, where appropriate, of the relevant passages | Relevant to claim No. |
|---|---|---|
| Y | KR 10-2018-0018405 A (SEASUNBIO MATERIALS) 21 February 2018<br>See abstract; tables 1-6; claims 1-33. | 1-20 |
| Y | KAESLER, S. et al. Toll-like receptor 2 ligands promote chronic atopic dermatitis through IL-4-mediated suppression of IL-10. Journal of Allergy and Clinical Immunology. 2014, vol. 134, pages 92-99<br>See abstract; page 93, left column, 98. | 1-20 |
| Y | US 2018-0312542 A1 (PRESIDENT AND FELLOWS OF HARVARD COLLEGE) 01 November 2018<br>See abstract; sequence identifier no. 5; claim 1. | 8-10 |
| A | JP 2008-220366 A (NATIONAL INSTITUTE OF ADVANCED INDUSTRIAL & TECHNOLOGY et al.) 25 September 2008<br>See abstract; claims 1-2, 6. | 1-20 |
| PX | KR 10-2019-0096148 A (SEASUN THERAPEUTICS) 19 August 2019<br>See abstract; claims 1-36. | 1-20 |
| PX | KR 10-2019-0096149 A (SEASUN THERAPEUTICS) 19 August 2019<br>See abstract; claims 1-23. | 1-20 |

☐ Further documents are listed in the continuation of Box C.  ☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 29 JANUARY 2020 (29.01.2020) | **29 JANUARY 2020 (29.01.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | |
| | Telephone No. |

Form PCT/ISA/210 (second sheet) (January 2015)

INTERNATIONAL SEARCH REPORT
Information on patent family members

International application No.

**PCT/KR2019/013970**

| Patent document cited in search report | Publication date | Patent family member | Publication date |
|---|---|---|---|
| KR 10-2018-0018405 A | 21/02/2018 | AU 2017-310146 A1 | 28/03/2019 |
| | | CA 3033474 A1 | 15/02/2018 |
| | | CN 109804070 A | 24/05/2019 |
| | | EP 3498844 A1 | 19/06/2019 |
| | | JP 2019-526624 A | 19/09/2019 |
| | | KR 10-1963885 B1 | 01/04/2019 |
| | | KR 10-2018-0100523 A | 11/09/2018 |
| | | US 2019-0185519 A1 | 20/06/2019 |
| | | WO 2018-030789 A1 | 15/02/2018 |
| US 2018-0312542 A1 | 01/11/2018 | WO 2017-070182 A1 | 27/04/2017 |
| JP 2008-220366 A | 25/09/2008 | None | |
| KR 10-2019-0096148 A | 19/08/2019 | WO 2019-156365 A1 | 15/08/2019 |
| KR 10-2019-0096149 A | 19/08/2019 | WO 2019-156366 A1 | 15/08/2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 2017008636 W
- US 4235871 A
- US 4501728 A
- US 4837028 A
- US 5019369 A

### Non-patent literature cited in the description

- **KOLE R. et al.** *Nature Rev. Drug Discov.,* 2012, vol. 11, 125-140
- **WILSON C. et al.** *Curr. Opin. Chem. Bio.,* 2006, vol. 10, 607-614
- **JOERGENSEN M. et al.** *Oligonucleotides,* 2011, vol. 21, 29-37
- **COUTO L. B.** *Curr. Opin. Pharmacol.,* 2010, vol. 5, 534-542
- **ZHI D. et al.** *Bioconjug. Chem.,* 2013, vol. 24, 487-519
- **BUYENS K. et al.** *J. Control Release,* 2012, vol. 158, 362-70
- **ROSSI, J. J. et al.** *Gene Ther.,* 2006, vol. 13, 583-584
- **YOUSEFI A. et al.** *J. Control Release,* 2013, vol. 170, 209-18
- **TRABULO S. et al.** *Curr. Pharm. Des.,* 2013, vol. 19, 2895-923
- **LADEMANN et al.** *Eur. J. Pharm. Biopharm.,* May 2007, vol. 66 (2), 159-64
- **CHEN et al.** *J. Pharmacol. Exp. Ther.,* November 2006, vol. 319 (2), 765-75
- **CHEN et al.** *Nat. Biotechnol.,* April 2006, vol. 24 (4), 455-60
- **D. W. PACK ; A. S. HOFFMAN ; S. PUN ; P. S. STAYTON.** Design and development of polymers for gene delivery. *Nat. Rev. Drug. Discov.,* 2005, vol. 4, 581-593
- **SZOKA et al.** *Ann. Rev. Biophys. Bioeng.,* 1980, vol. 9, 467

<110>    SEASUN THERAPEUTICS

<120>    Composition for Preventing or Treating Atopic Dermatitis
         Comprising Skin Permeable Nucleic Acid Complex

<130>    PP-B2288

<150>    KR 10-2018-0135892
<151>    2018-11-07

<160>    19

<170>    KoPatentIn 3.0

<210>    1
<211>    17
<212>    DNA
<213>    Artificial Sequence

<220>
<223>    BNA1


<400>    1
agtgcgcatt ggtctat                                                    17


<210>    2
<211>    17
<212>    DNA
<213>    Artificial Sequence

<220>
<223>    BNA2


<400>    2
atgtaggtga tcctgtt                                                    17


<210>    3
<211>    15
<212>    DNA
<213>    Artificial Sequence

<220>
<223>    BNA3


<400>    3
agtagtcgcg tatgt                                                      15


<210>    4
<211>    15
<212>    DNA
<213>    Artificial Sequence

<220>
<223>    BNA4


<400>    4

```
aagccacccc attgg                                                      15


<210>      5
<211>      17
<212>      DNA
<213>      Artificial Sequence

<220>
<223>      CPNA5


<400>      5
atagaccaat gcgcact                                                    17


<210>      6
<211>      5
<212>      DNA
<213>      Artificial Sequence

<220>
<223>      CPNA6


<400>      6
aacag                                                                  5


<210>      7
<211>      5
<212>      DNA
<213>      Artificial Sequence

<220>
<223>      CPNA7


<400>      7
gacaa                                                                  5


<210>      8
<211>      5
<212>      DNA
<213>      Artificial Sequence

<220>
<223>      CPNA8


<400>      8
aacag                                                                  5


<210>      9
<211>      5
<212>      DNA
<213>      Artificial Sequence

<220>
<223>      CPNA9
```

<400>    9
gacaa                                                                          5


<210>    10
<211>    17
<212>    DNA
<213>    Artificial Sequence

<220>
<223>    CPNA10


<400>    10
aacaggatca cctacat                                                             17


<210>    11
<211>    17
<212>    DNA
<213>    Artificial Sequence

<220>
<223>    CPNA11


<400>    11
tacatccact aggacaa                                                             17


<210>    12
<211>    17
<212>    DNA
<213>    Artificial Sequence

<220>
<223>    CPNA12


<400>    12
aacaggatca cctacat                                                             17


<210>    13
<211>    17
<212>    DNA
<213>    Artificial Sequence

<220>
<223>    CPNA13


<400>    13
tacatccact aggacaa                                                             17


<210>    14
<211>    15
<212>    DNA
<213>    Artificial Sequence

<220>

<223>    CPNA14


<400>    14
tcatcagcgc ataca                                                                15


<210>    15
<211>    5
<212>    DNA
<213>    Artificial Sequence

<220>
<223>    CPNA15


<400>    15
ccaat                                                                            5


<210>    16
<211>    5
<212>    DNA
<213>    Artificial Sequence

<220>
<223>    CPNA16


<400>    16
taacc                                                                            5


<210>    17
<211>    15
<212>    DNA
<213>    Artificial Sequence

<220>
<223>    CPNA17


<400>    17
ccaatggggt ggctt                                                                15


<210>    18
<211>    15
<212>    DNA
<213>    Artificial Sequence

<220>
<223>    CPNA18


<400>    18
ttcggtgggg taacc                                                                15


<210>    19
<211>    13
<212>    PRT
<213>    Artificial Sequence

```
<220>
<223>    linker19


<400>    19
Gly Leu Phe Asp Ile Ile Lys Lys Ile Ala Glu Ser Phe
 1               5                   10
```